# DEMANDE DE BREVET EUROPEEN

(11) **EP 3 461 883 A1**
(43) Date de publication de la demande: **03.04.2019**
(21) Numéro de dépôt: 18207005.2
(22) Date de dépôt: 29.11.2013
(51) Int. Cl.: C12N 1/16, C12N 1/18, C12R 1/85, C12R 1/865, C40B 40/02, C40B 50/06

(54) **PROCÉDÉ D'AMÉLIORATION DE SOUCHES DE LEVURE**

(30) Priorité: 30.11.2012 FR 1261456
(62) Demande divisionnaire de: 13799018.0
(71) Demandeur: Institut Curie, 75005 Paris (FR); CENTRE NATIONAL DE LA RECHERCHE SCIENTIFIQUE, 75016 Paris (FR); Université De Nice Sophia Antipolis, 06103 Nice (FR); INSERM (Institut National de la Santé et de la Recherche Médicale), 75013 Paris (FR); Sorbonne Université, 75006 Paris (FR)
(72) Inventeur: NICOLAS, Alain, 75015 Paris (FR); LITI, Gianni, 06300 Nice (FR)
(74) Mandataire: Cabinet Becker et Associés

(57) **Abrégé**

La présente invention concerne un procédé pour améliorer une souche de levure d'intérêt industriel, en particulier une souche hybride stérile, sans avoir recours aux techniques d'ADN recombinant.

## Description

La présente invention relève du domaine de la microbiologie, plus particulièrement du domaine des levures. Elle concerne notamment un procédé pour améliorer ou modifier une souche de levure d'intérêt industriel, en particulier une souche hybride incapable de sporuler, sans avoir recours à des techniques d'ADN recombinant.

### ARRIERE-PLAN TECHNOLOGIQUE DE L'INVENTION

Les levures trouvent leur application dans des domaines industriels extrêmement variés. Du fait de l'innocuité d'un grand nombre d'espèces, les levures sont notamment utilisées dans l'industrie alimentaire comme agent de fermentation en boulangerie, en brasserie, vinification ou distillerie, ou sous forme d'extraits comme éléments nutritionnels ou agents de sapidité. Elles peuvent également trouver leur usage dans la production industrielle de bioéthanol ou de molécules d'intérêt telles que des vitamines, des antibiotiques, des vaccins, des enzymes ou des hormones stéroïdiennes, ou encore dans les procédés de dégradation des matières cellulosiques.

La diversité des applications industrielles des levures, et en particulier de *Saccharomyces cerevisiae*, implique qu'il existe une demande constante pour des souches de levure présentant des caractéristiques améliorées ou, tout au moins, adaptées à une nouvelle utilisation ou de nouvelles conditions de culture.

Afin d'obtenir une souche présentant une caractéristique particulière, l'homme du métier dispose d'une variété d'outils et de méthodes. Il peut notamment procéder à la modification génétique de la souche en y introduisant un ou plusieurs gènes hétérologues, en modifiant ou supprimant l'expression de gènes endogènes. Cependant, la modification génétique d'une souche par une technique d'ADN recombinant peut constituer un frein à son exploitation que ce soit pour des raisons réglementaires, sanitaires ou environnementales.

La reproduction sexuée peut également être utilisée pour l'amélioration des levures en croisant deux souches parentales présentant des caractéristiques intéressantes et en sélectionnant une souche hybride apportant la combinaison recherchée des caractéristiques parentales. Les cellules haploïdes issues des produits de sporulation peuvent aussi être criblées afin d'identifier celles dans lesquelles la recombinaison méiotique a permis d'obtenir la caractéristique recherchée.

Cependant, les souches de levure utilisées dans l'industrie sont souvent des cellules hybrides obtenues par le croisement de souches génétiquement distinctes et peuvent être stériles du fait de leur incapacité à produire des spores viables. Pour ces cellules hybrides qui présentent un intérêt économique majeur, il est donc impossible de générer de la diversité génétique par reproduction sexuée et criblage des produits de sporulation.

### RESUME DE L'INVENTION

L'objectif de la présente invention est de fournir un nouveau procédé pour améliorer des souches de levure d'intérêt industriel, dont des levures hybrides stériles, sans avoir recours aux techniques d'ADN recombinant, et pour obtenir des levures, de préférence avec un degré de ploïdie supérieur ou égal à 2, et en particulier diploïdes, de génotype recombiné permettant l'analyse rapide de caractères quantitatifs d'intérêt.

Selon un premier aspect, la présente invention concerne donc un procédé pour améliorer une souche de levure d'intérêt industriel, comprenant :
a) le transfert de la levure d'un milieu riche à un milieu de sporulation, de préférence sans source de carbone fermentescible ni d'azote ;
b) l'incubation des levures dans le milieu de sporulation durant un temps suffisant pour induire la formation des cassures double-brin Spo 11-dépendantes ;
c) la mise en contact des levures avec une source de carbone et d'azote avant la ségrégation réductionnelle des chromosomes de la première division de méiose afin d'obtenir des levures recombinées ;
d) la récupération des levures recombinées ; et
e) le criblage ou la sélection des levures recombinées afin d'identifier celles présentant l'amélioration recherchée.

Le procédé peut comprendre en outre l'obtention d'une ou des levures recombinées présentant l'amélioration recherchée à partir du criblage ou de la sélection de l'étape e).

Elle concerne également l'utilisation du processus RTG de la levure pour améliorer une souche de levure d'intérêt industriel, ledit processus RTG étant induit par le transfert de la levure d'un milieu riche à un milieu de sporulation, de préférence sans source de carbone fermentescible ni d'azote, l'incubation des levures dans le milieu de sporulation durant un temps suffisant pour induire la formation des cassures double-brin Spo11-dépendantes, et la mise en contact des levures avec une source de carbone et d'azote avant la ségrégation réductionnelle des chromosomes de la première division de méiose.

De préférence, la levure d'intérêt industriel présente un degré de ploïdie supérieur ou égal à 2.

La souche de levure d'intérêt industriel peut être une levure hybride.

Elle peut être haploïde, aneuploïde, diploïde ou polyploïde, de préférence diploïde, aneuploïde ou polyploïde, et de manière plus particulièrement préférée diploïde.

De préférence, la levure d'intérêt industriel est une levure hybride diploïde.

La souche de levure d'intérêt industriel peut être une levure stérile, de préférence, une souche hybride stérile.

Selon un mode préféré, la souche de levure d'intérêt industriel est une souche hybride diploïde stérile.

Selon un mode préféré, la souche est un organisme non génétiquement modifié.

Les levures recombinées présentent un ou plusieurs évènements de recombinaison par cellule, de préférence plusieurs événements de recombinaison. De préférence, lesdits évènements de recombinaison induisent une diminution du taux d'hétérozygotie.

Un acide nucléique codant pour une protéine de fusion, sous le contrôle d'un promoteur, de préférence spécifique de la méiose, comprenant un domaine de liaison à l'ADN lié de façon opérationnelle à une protéine Spo11 ou à une protéine partenaire de Spo11, peut être introduit dans la souche d'intérêt industriel afin d'augmenter localement la fréquence des cassures double-brin en prophase de méiose ou de modifier la distribution desdites cassures le long des chromosomes.

Les étapes a) à d) ou a) à e) peuvent être répétées au moins une fois à partir d'une ou plusieurs levures recombinées.

Les levures recombinées récupérées à l'étape d) peuvent être conservées sous forme de banques de levures avant d'être criblées ou sélectionnées à l'étape e).

De préférence, les levures recombinées ou améliorées obtenues par le procédé selon l'invention ne sont pas des organismes génétiquement modifiés (OGM).

La souche de levure d'intérêt industriel est de préférence destinée à l'industrie alimentaire, à la production de biocarburant, en particulier de bioéthanol, à la production de molécules d'intérêt, telles que des vitamines, des antibiotiques, des vaccins, des enzymes ou des hormones stéroïdiennes, ou à la dégradation de la cellulose ou de biomasse lignocellulosique et/ou appartient, de préférence, à l'espèce *Saccharomyces cerevisiae* ou est un hybride obtenu à partir de *Saccharomyces cerevisiae*.

L'amélioration recherchée peut concerner une ou plusieurs caractéristiques sélectionnées dans le groupe constitué de la vitesse de croissance, la thermotolérance, la cryotolérance, la sensibilité au pH, la capacité de fermentation, la rapidité de fermentation, la résistance à l'éthanol, la résistance à un composé particulier présent dans le milieu de fermentation ou excrété par la culture cellulaire, la morphologie cellulaire, le caractère de floculation, la sensibilité à une molécule particulière, l'efficacité de sporulation, les profils aromatiques, les exigences nutritionnelles, la résistance au séchage, et la fermentation d'un sucre particulier.

Selon un second aspect, la présente invention concerne un procédé pour générer une banque de levures recombinées à partir d'une levure, comprenant :
a) le transfert de la levure d'un milieu riche à un milieu de sporulation, de préférence sans source de carbone fermentescible ni d'azote ;
b) l'incubation des levures dans le milieu de sporulation durant un temps suffisant pour induire des cassures double-brin Spo 11-dépendantes :
(c) la mise en contact des levures avec une source de carbone et d'azote avant la ségrégation réductionnelle des chromosomes de la première division de méiose afin d'obtenir des levures recombinées ;
d) la récupération des levures recombinées afin de former une banque de levures recombinées.

La levure peut être haploïde, aneuploïde, diploïde ou polyploïde, de préférence diploïde, aneuploïde ou polyploïde, et de manière plus particulièrement préférée diploïde.

De préférence, la levure présente un degré de ploïdie supérieur ou égal à 2.

Elle concerne également une banque de levures recombinées obtenues selon ledit procédé.

Selon un autre aspect, elle concerne aussi un procédé pour identifier ou localiser l'information génétique codant pour une caractéristique d'intérêt, de préférence un caractère quantitatif d'intérêt (QTL), dans une levure, de préférence une levure présentant un degré de ploïdie supérieur ou égal à 2, comprenant :
a) le transfert de la levure d'un milieu riche à un milieu de sporulation, de préférence sans source de carbone fermentescible ni d'azote ;
b) l'incubation des levures dans le milieu de sporulation durant un temps suffisant pour induire des cassures double-brin Spo 11-dépendantes ;
c) la mise en contact des levures avec une source de carbone et d'azote avant la ségrégation réductionnelle des chromosomes de la première division de méiose afin d'obtenir des levures recombinées ;
d) la récupération des levures recombinées ; et
e) l'analyse des génotypes et phénotypes des levures recombinées afin identifier ou localiser l'information génétique codant pour la caractéristique d'intérêt.

En particulier, la caractéristique d'intérêt peut être sélectionnée dans le groupe constitué de la vitesse de croissance, la thermotolérance, la cryotolérance, la sensibilité au pH, la capacité de fermentation, la rapidité de fermentation, la résistance à l'éthanol, la résistance à un composé particulier présent dans le milieu de fermentation ou excrété par la culture cellulaire, la morphologie cellulaire, le caractère de floculation, la sensibilité à une molécule particulière, l'efficacité de sporulation, les profils aromatiques, les exigences nutritionnelles, la résistance au séchage, et la fermentation d'un sucre particulier.

### BREVE DESCRIPTION DES DESSINS

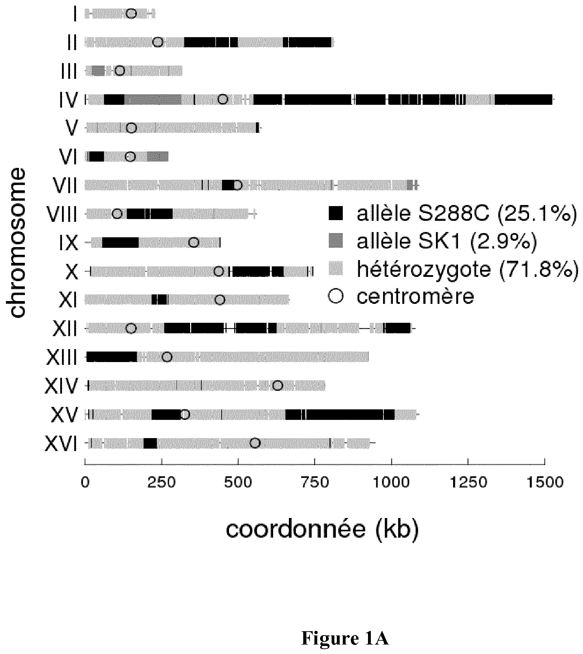

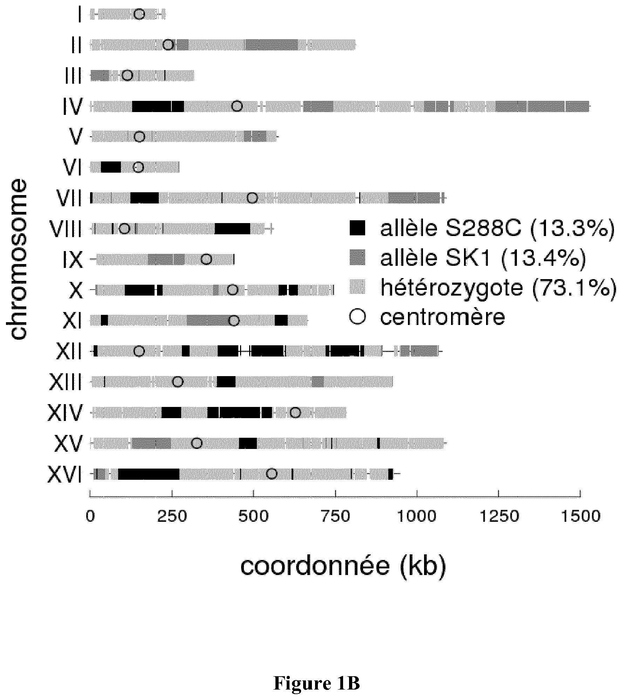

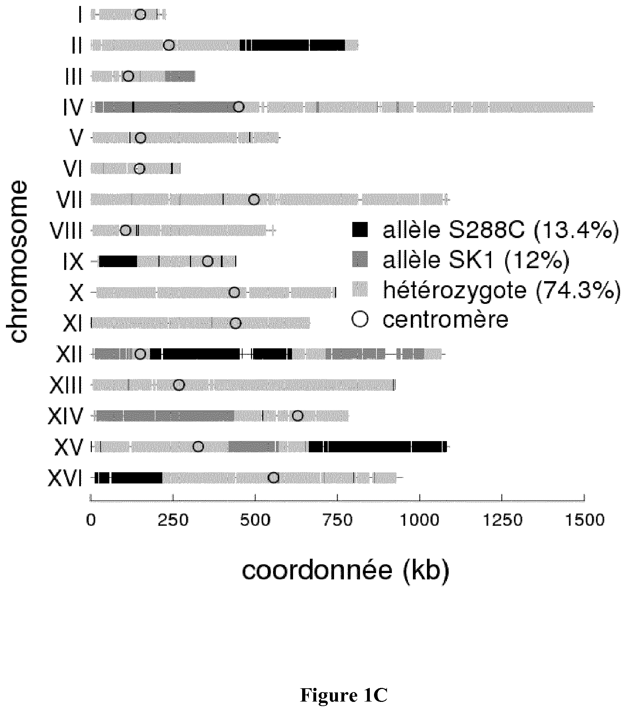

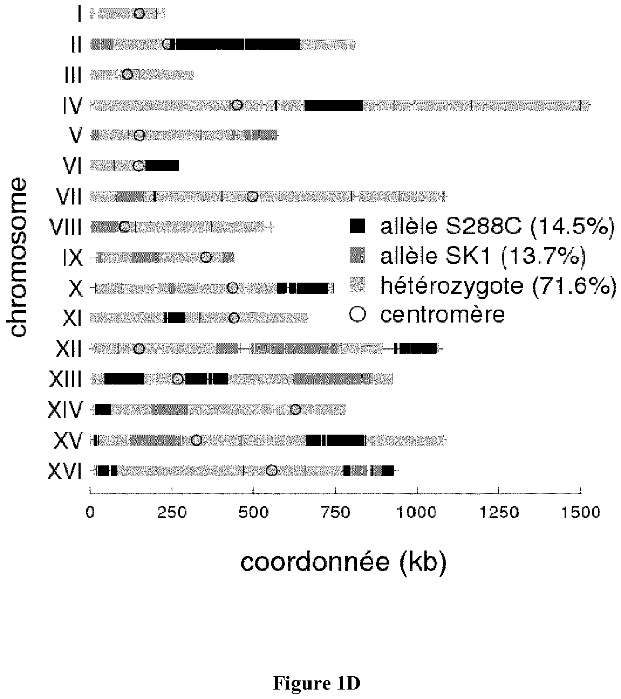

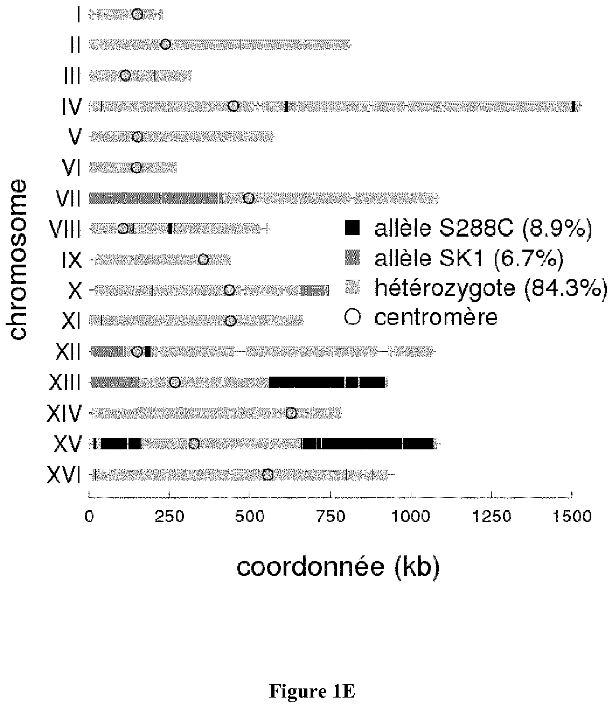

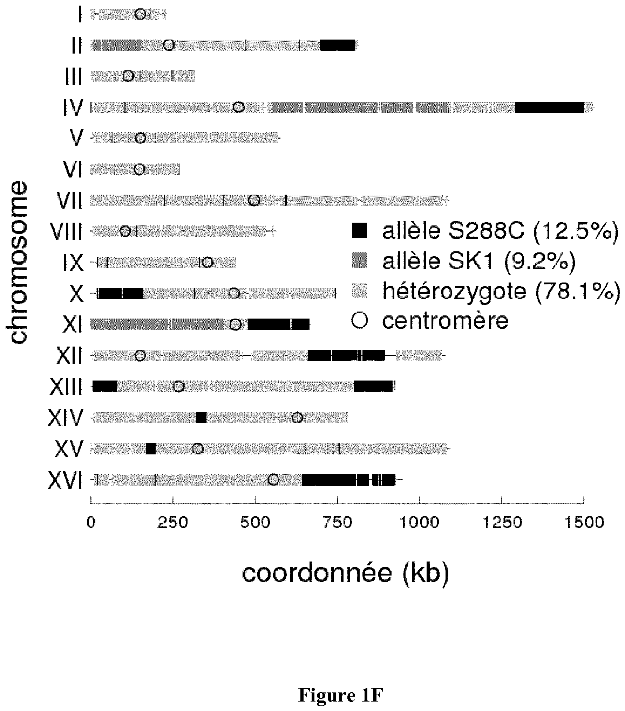

**Figure 1**: Cartographie des SNPs dans les cellules RTG obtenue par le protocole 2 de « return-to-growth », c'est-à-dire en déposant les cellules prélevées au cours de la sporulation sur un milieu sélectif DO-arginine afin de sélectionner les cellules recombinantes porteuses d'un allèle ARG4. Pour chaque diploïde RTG, le génotype des deux chromosomes homologues est représenté: hétéroallélique (gris clair), mono-allélique (gris foncé) si d'origine SK1 ou mono-allélique (noir) si d'origine S288C. ***Fig*. *1A*** : Génotype de la cellule RTG AND1708 (21 régions mono-alléliques de plus de 20 kb ; 34 jonctions entre régions mono-alléliques et régions bi-alléliques, ou entre régions mono-alléliques d'allèles différents ; taille de la plus grande région de perte d'hétérozygotie : environ 691kb). ***Fig*. *1B*** : Génotype de la cellule RTG AND1709 (29 régions mono-alléliques de plus de 20 kb ; 53 jonctions entre régions mono-alléliques et régions bi-alléliques, ou entre régions mono-alléliques d'allèles différents ; taille de la plus grande région de perte d'hétérozygotie : environ 264kb). ***Fig. 1C*** : Génotype de la cellule RTG AND1710 (11 régions mono-alléliques de plus de 20 kb ; 15 jonctions entre régions mono-alléliques et régions bi-alléliques, ou entre régions mono-alléliques d'allèles différents ; taille de la plus grande région de perte d'hétérozygotie : environ 431kb). ***Fig. 1D*****:** Génotype de la cellule RTG AND1711 (25 régions mono-alléliques de plus de 20 kb ; 38 jonctions entre régions mono-alléliques et régions bi-alléliques, ou entre régions mono-alléliques d'allèles différents ; taille de la plus grande région de perte d'hétérozygotie : environ 398kb). ***Fig. 1E*** : Génotype de la cellule RTG AND1712 (7 régions mono-alléliques de plus de 20 kb ; 7 jonctions entre régions mono-alléliques et régions bi-alléliques, ou entre régions mono-alléliques d'allèles différents ; taille de la plus grande région de perte d'hétérozygotie : environ 415kb). ***Fig. 1F*** : Génotype de la cellule RTG AND1720 (13 régions mono-alléliques de plus de 20 kb ; 18 jonctions entre régions mono-alléliques et régions bi-alléliques, ou entre régions mono-alléliques d'allèles différents ; taille de la plus grande région de perte d'hétérozygotie : environ 537kb).

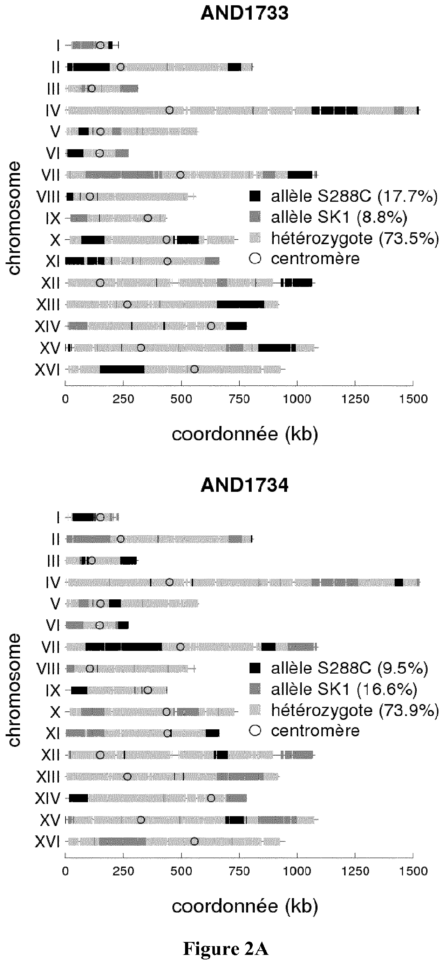

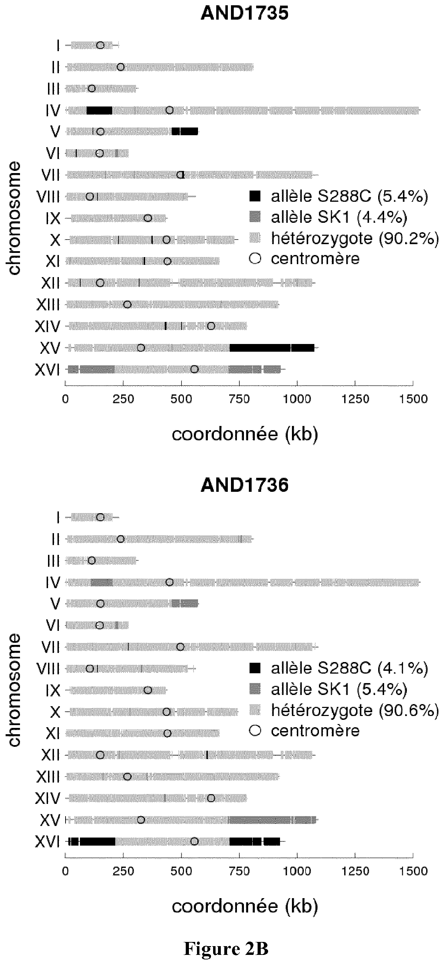

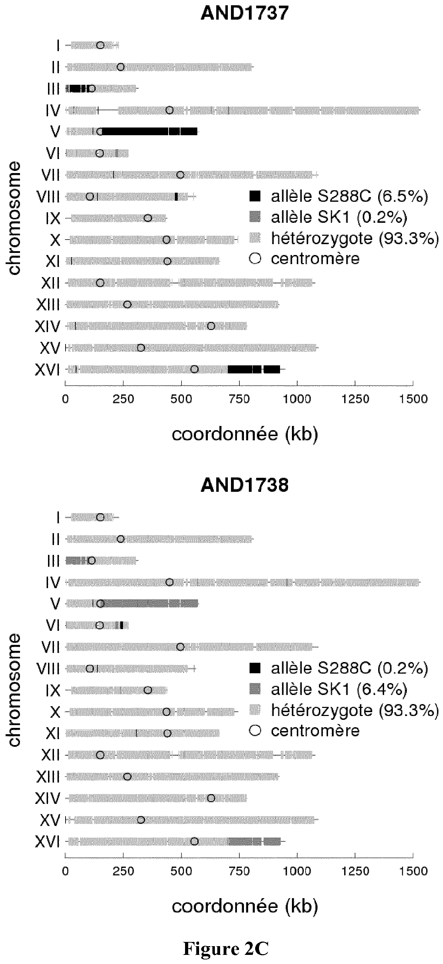

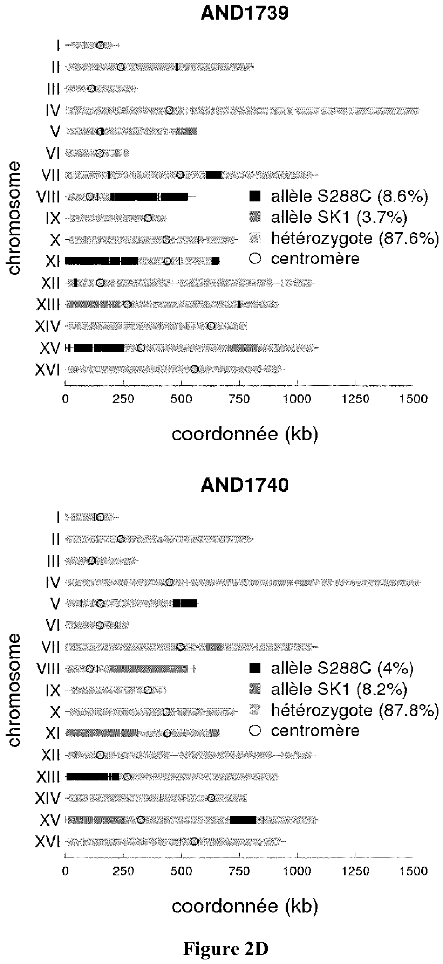

**Figure 2**: Cartographie des SNPs dans les couples mère/filles issus du protocole 3 de « return-to-growth », c'est-à-dire par isolement des cellules RTG par micromanipulation. Pour chaque diploïde RTG, les deux cellules mère et fille provenant du même événement de RTG sont regroupées. ***Fig. 2A*** : Génotype des cellules RTG AND1733 (cellule mère) et AND1734 (cellule fille) (29 régions mono-alléliques de plus de 20 kb ; 45 jonctions entre régions mono-alléliques et régions bi-alléliques, ou entre régions mono-alléliques d'allèles différents ; taille de la plus grande région de perte d'hétérozygotie : environ 300kb). ***Fig. 2B*** : Génotype des cellules RTG AND1735 (cellule mère) et AND1736 (cellule fille) (5 régions mono-alléliques de plus de 20 kb ; 6 jonctions entre régions mono-alléliques et régions bi-alléliques, ou entre régions mono-alléliques d'allèles différents ; taille de la plus grande région de perte d'hétérozygotie : environ 361kb). ***Fig. 2C*** : Génotype des cellules RTG AND1737 (cellule mère) et AND1738 (cellule fille) (3 régions mono-alléliques de plus de 20 kb ; 3 jonctions entre régions mono-alléliques et régions bi-alléliques, ou entre régions mono-alléliques d'allèles différents ; taille de la plus grande région de perte d'hétérozygotie : environ 393kb). ***Fig. 2D*** : Génotype des cellules RTG AND1739 (cellule mère) et AND1740 (cellule fille) (8 régions mono-alléliques de plus de 20 kb ; 10 jonctions entre régions mono-alléliques et régions bi-alléliques, ou entre régions mono-alléliques d'allèles différents ; taille de la plus grande région de perte d'hétérozygotie : environ 357kb).

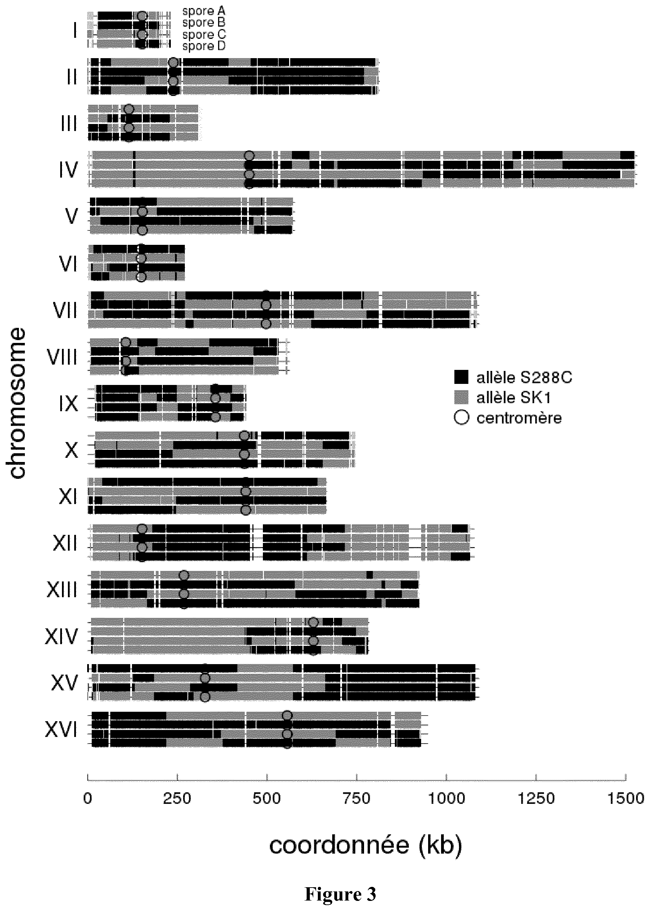

**Figure 3****.** Génotype des quatre spores de la tétrade issue de la cellule RTG AND1710. Pour chacun des 16 chromosomes, le génotype des spores A, B, C et D de la tétrade est représenté l'un en dessous de l'autre.

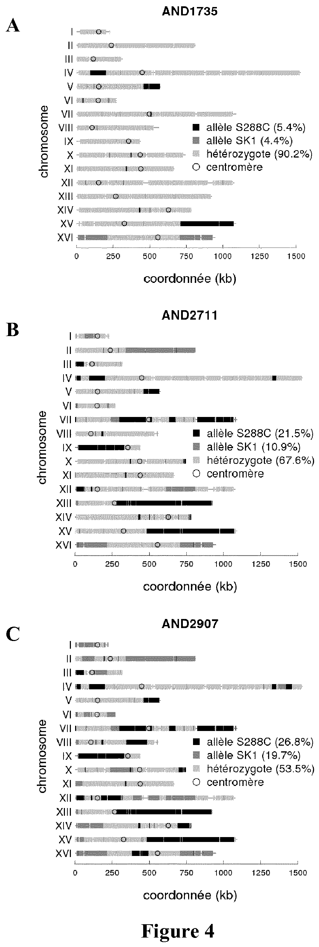

**Figure 4****.** Cartographie des SNPs des cellules issues de la réitération du processus RTG. La cellule RTG AND1735 (Figure 2B et Figure 4A) a subi un second cycle de RTG générant la cellule diploïde recombinée AND2711 (Figure 4B) puis un troisième cycle de RTG générant la cellule diploïde recombinée AND2907 (Figure 4C). Le pourcentage d'hétérozygotie et le pourcentage relatif des allèles de fond génétique S288c ou SK1 calculé parmi les régions de perte d'hétérozygotie sont indiqués (Fig. 4A-C). La réitération du processus RTG conduit à des recombinaisons additionnelles, une diminution graduelle du degré d'hétérozygotie et des variations dans la proportion des régions mono-alléliques de génotype purement S288C et SK1 (Fig.4 A-C).

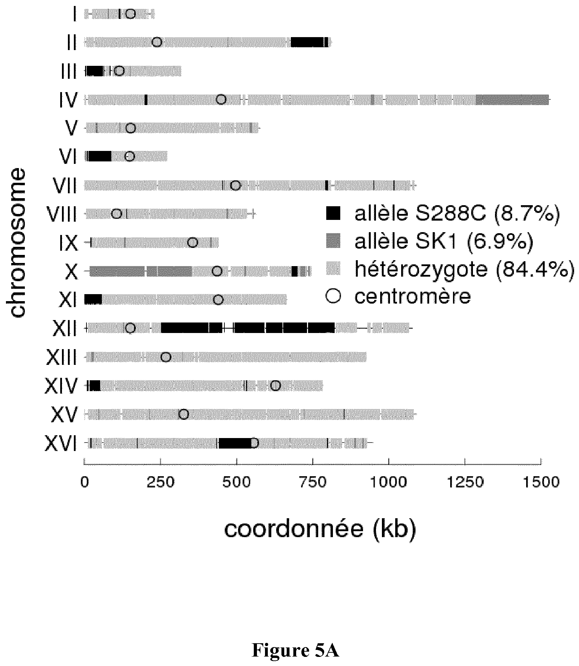

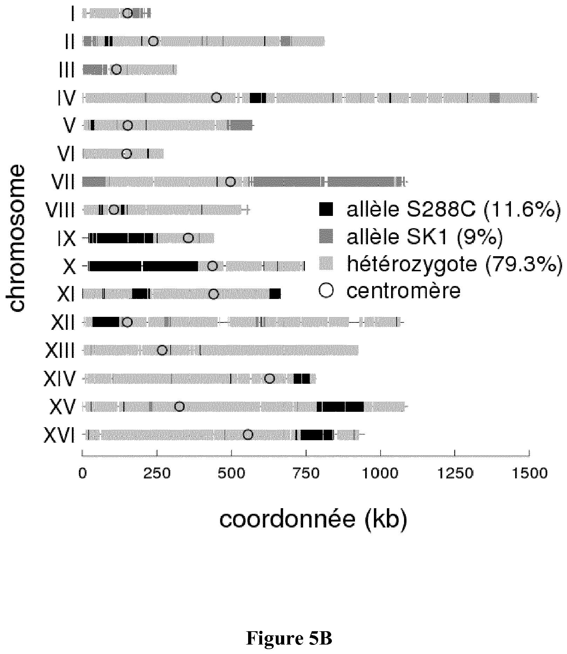

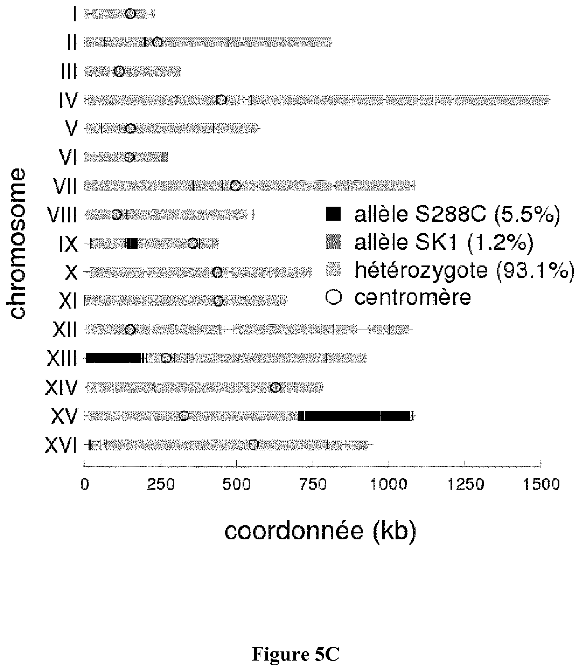

**Figure 5****.** Cartographie des SNPs des cellules RTG issues de la souche AND2248 déficiente en sporulation. Ces trois cellules AND2642 (***Fig. 5A***), AND2652 (***Fig. 5B***)*,* et AND2658 (***Fig. 5C***) issues d'un cycle de RTG sont diploïdes et de génotype recombiné. Leur génotype est différent. Dans les trois cas, le degré d'hétérozygotie par rapport à la cellule parentale s'est réduit (84,4%, 79,3% et 93,1%) et des régions mono-alléliques S288C ou SK1 sont apparues en proportion variables.

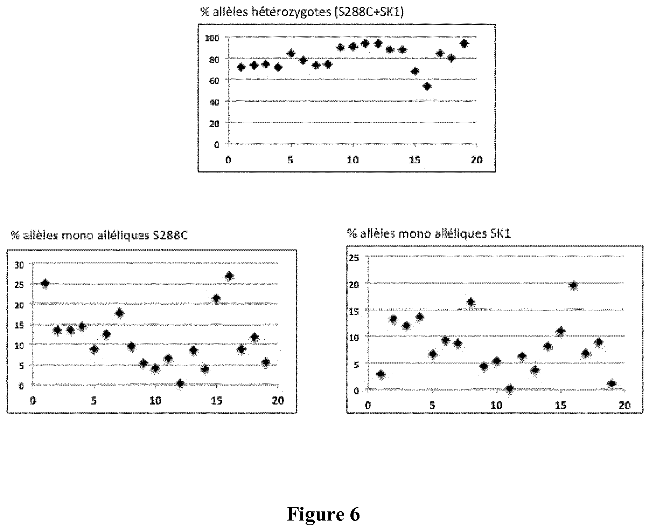

**Figure 6****.** Analyse comparative du génotype allélique de 19 cellules RTG issues des cellules diploïdes AND1702, AND1735, AND2907 et AND2248. Pour chaque cellule RTG, le pourcentage de SNPs bi-alléliques (hétérozygotes), mono-allélique si d'origine 288C ou si mono allélique d'origine SK1 sont représentés. Leurs proportions varient d'une cellule RTG à l'autre.

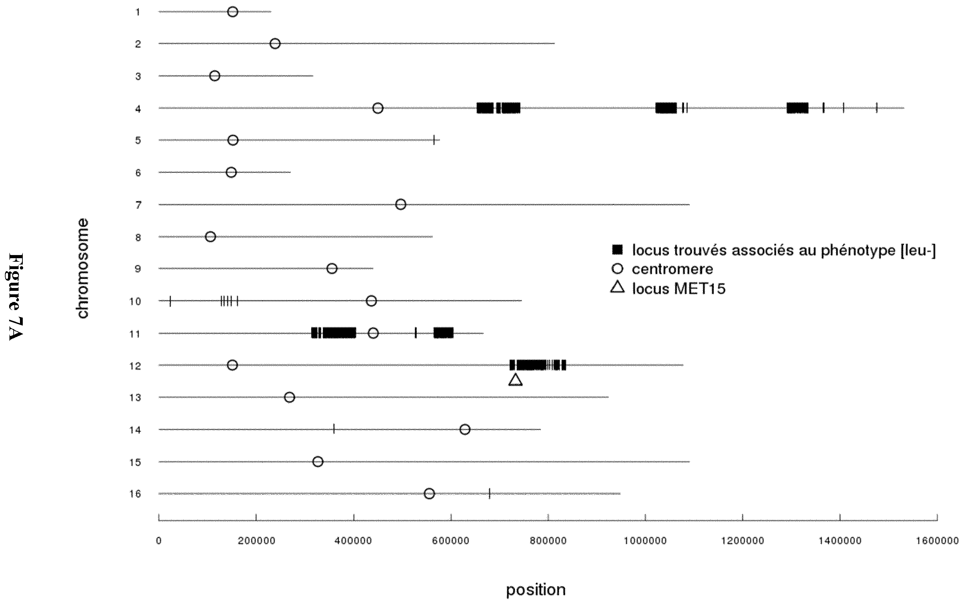

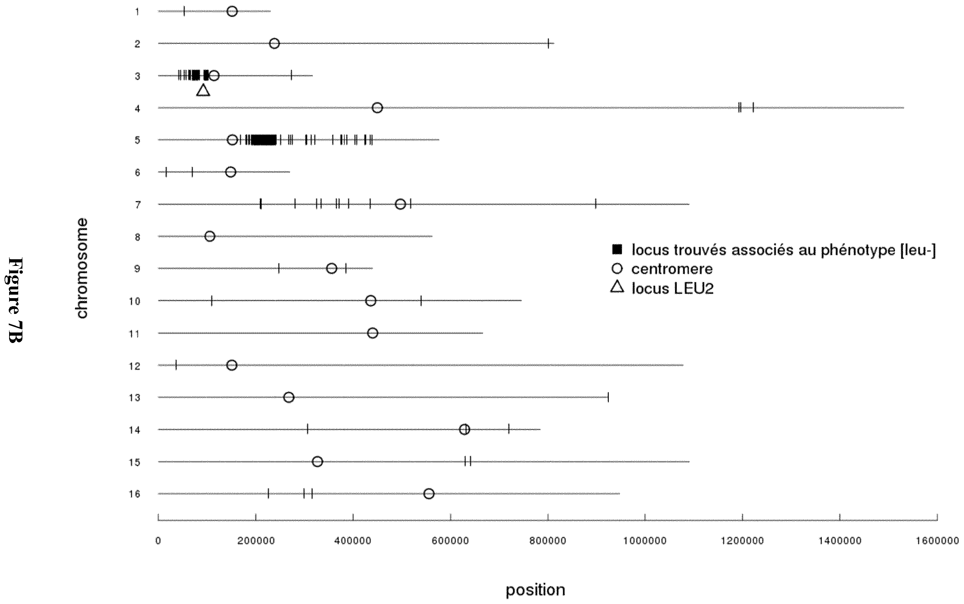

**Figure 7****.** Cartographie des positions de polymorphisme SNP associées à des phénotypes simples. La longueur de chaque chromosome est représentée par une fine ligne noire horizontale. L'étude de plusieurs cellules issues du RTG a pour effet de réduire le nombre de régions candidates et d'améliorer la résolution de la cartographie dans les régions restantes. En raison du faible nombre d'échantillons analysés, plusieurs régions candidates se trouvent associées au phénotype d'auxotrophie pour la méthionine ou la leucine. ***Fig. 7A*** : Cartographie des positions de polymorphisme SNP associées au phénotype d'auxotrophie pour la méthionine. Le locus MET15 (triangle) est trouvé associé au phénotype d'auxotrophie pour la méthionine. ***Fig. 7B*** : Cartographie des positions de polymorphisme SNP associées au phénotype d'auxotrophie pour la leucine. Le locus LEU2 (triangle) est trouvé associé au phénotype d'auxotrophie pour la leucine.

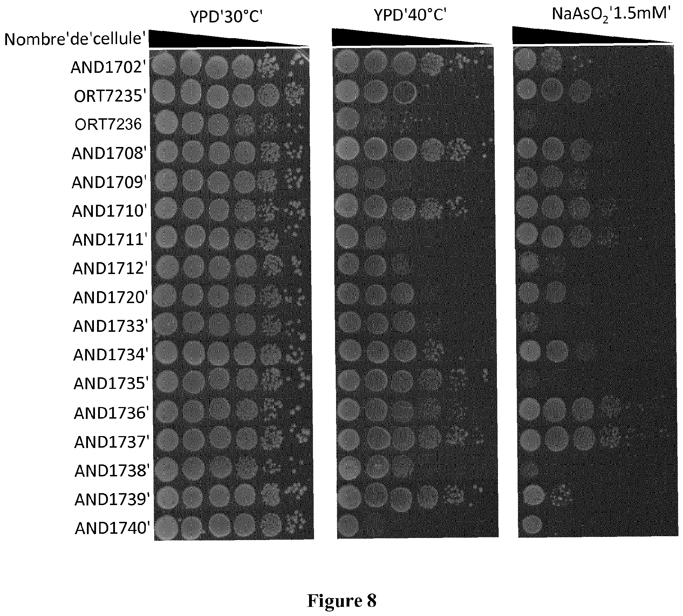

**Figure 8****.** Tests de croissance des cellules parentales et RTG. Croissance à 30°C, 40°C sur milieu YPD et à 30°C sur milieu YPD contenant de l'arsenite de sodium (1,5mM NaAs0₂). Pour chaque souche, 10⁶, 10⁵, 10⁴, 10³, 10², et 10¹ cellules ont été déposées sur milieu YPD et incubées 2 jours à 30°C avec et sans NaAs0₂ ou 40°C (sans NaAS0₂).

### DESCRIPTION DETAILLEE DE L'INVENTION

Parmi les différentes espèces de levures exploitées dans l'industrie, *Saccharomyces cerevisiae* est fréquemment utilisée. *S cerevisiae* est un organisme eucaryote unicellulaire haplodiplobiontique, c'est-à-dire un organisme présentant un cycle de reproduction au cours duquel la multiplication cellulaire se fait à la fois dans un état haploïde et un état diploïde.

Les cellules peuvent se reproduire de manière clonale par des divisions mitotiques. La cellule fille apparaît alors sous la forme d'un bourgeon dès la phase S de réplication du génome, puis grossit et se sépare de la cellule mère. Lors des divisions mitotiques, l'ensemble du génome (12 071 326 nucléotides pour le génome nucléaire) est répliqué et transmis de façon fidèle de la cellule mère à la cellule fille. Des phénomènes de recombinaison homologue peuvent exister mais essentiellement pour assurer la réparation des cassures double-brins accidentelles de l'ADN qui peuvent se produire lors du blocage de la réplication ou lors de l'exposition des cellules à des agents génotoxiques. Dans ce cas, la chromatide soeur est préférentiellement utilisée comme matrice. Lors de la mitose, la recombinaison entre les chromosomes homologues est rare ce qui permet aux cellules en croissance végétative de maintenir le même patrimoine génétique.

Les levures peuvent également se reproduire par reproduction sexuée à travers la méiose et la sporulation. D'une part, deux cellules haploïdes de types sexuels opposés (MATa et MATα) peuvent fusionner pour former une cellule diploïde MATa/MATα qui se reproduit à l'identique par mitose. D'autre part, dans des conditions de carence nutritionnelle, les cellules diploïdes entrent en sporulation en passant par un processus méiotique qui génère, pour chaque cellule diploïde, quatre spores haploïdes empaquetées dans une tétrade. La germination de ces spores produit des cellules haploïdes qui seront à leur tour capables de fusionner pour former une nouvelle cellule diploïde.

L'initiation du cycle méiotique dépend de plusieurs signaux transmis simultanément à la cellule : un signal génétique de diploïdie indiqué par la présence des deux allèles de type sexuel MATa et MATα, et des signaux nutritionnels d'absence de source d'azote et de carbone fermentescible et de présence d'une source de carbone pouvant être métabolisé par respiration (Honigberg and Purnapatre, 2003).

Lors de la méiose, contrairement à ce qui se passe lors de divisions mitotiques, l'information génétique des cellules diploïdes parentales n'est pas transmise à l'identique aux cellules haploïdes. Celle-ci est en effet réduite de moitié et de nombreux échanges génétiques entre les chromosomes homologues ont lieu durant la prophase I de méiose. La recombinaison méiotique qui s'opère durant la prophase I implique (i) la formation de cassures double-brin par la protéine Spo11, (ii) la résection de nucléotides à l'extrémité 5' des cassures, (iii) l'invasion de l'extrémité 3'-sortante d'un brin au niveau d'une chromatide du chromosome homologue et (iv) la réparation de la cassure produisant un crossing-over (échange réciproque entre les chromosomes homologues), une conversion génique (copie d'un segment d'ADN sans échange réciproque entre les chromosomes homologues) ou les deux (conversion génique associée à un crossing-over).

Une particularité de la levure *S. cerevisiae* tient en ce que l'induction de l'entrée en prophase I de méiose est réversible. Ce processus, appelé « Return to Growth » (RTG), se produit lorsque les cellules diploïdes entrées en méiose en réponse à une carence nutritionnelle sont remises en présence d'une source de carbone et d'azote après la formation des cassures double-brin Spo11 dépendantes mais avant la première division de méiose (Honigberg and Esposito, 1994). Dans ces conditions, elles interrompent leur progression dans les stades de différenciation méiotique pour reprendre un mode de croissance mitotique tout en induisant des recombinaisons lors de la réparation des cassures double-brin provoquées par Spo11 (Sherman and Roman, 1963 ; Esposito and Esposito, 1974 ; Zenvirth et al., 1997). Lors du processus de RTG, il a été observé que les cassures double-brin sont très rapidement réparées après le transfert des cellules en milieu riche (Zenvirth et al., 1997). Bien que les mécanismes mis en jeu pour ces réparations ne soient pas encore élucidés, il a été démontré qu'ils sont distincts de ceux mis en place lors de la méiose et semblent minimiser la production de crossing-over afin de préserver l'intégrité du génome et de limiter les pertes d'hétérozygotie (Dayani et al., 2011).

Afin d'établir une cartographie précise des échanges génétiques entre chromosomes homologues lors du processus de RTG, les inventeurs ont procédé au séquençage complet des seize chromosomes de quatorze cellules diploïdes obtenues par RTG à partir d'une levure diploïde hybride dont les chromosomes homologues différaient de 63 901 marqueurs de polymorphisme nucléotidique (SNPs) (soit un marqueur tous les 187 pb en moyenne). Ils ont ainsi pu déterminer le nombre et la nature (crossing-over ou conversion génique) des évènements de recombinaison qui se sont opérés dans chacune des cellules durant ce processus.

Ils ont ainsi observé, de manière tout à fait inattendue, que les cellules issues du RTG étaient hautement recombinées (à raisons de 3 à 51 évènements de recombinaison par cellule) et se caractérisaient par une très grande diversité génétique des chromosomes homologues, aucune des cellules ne présentant le même profil de recombinaison. De plus, les inventeurs ont montré que les cellules issues de RTG présentaient des recombinaisons associés à des pertes d'hétérozygoties de longueur variable impliquant un ou plusieurs SNPs. La taille physique de la perte d'hétérozygotie variait entre 2 bp et 700 kb. Grâce à cette vision globale des événements de recombinaison à l'échelle du génome, les inventeurs ont démontré que, contrairement à ce qui avait été précédemment décrit, les mécanismes de réparation des cassures double-brin lors du processus RTG ne limitaient pas les pertes d'hétérozygotie mais généraient au contraire une diversification massive de l'information génétique du génome de la cellule hybride parentale.

Il est ainsi apparu de manière surprenante que le processus de RTG pouvait être utilisé pour générer une grande diversité génétique. Ce processus est donc particulièrement intéressant pour l'amélioration ou la modification des levures hybrides, notamment des levures hybrides stériles pour lesquelles il est impossible d'obtenir des génotypes recombinants à partir des spores.

Ainsi, la présente invention concerne un procédé pour améliorer une souche de levure d'intérêt industriel comprenant :
a) le transfert de la levure d'un milieu riche à un milieu de sporulation, de préférence sans source de carbone fermentescible ni d'azote ;
b) l'incubation des levures dans le milieu de sporulation durant un temps suffisant pour induire des cassures double-brin Spo 11-dépendantes ;
c) la mise en contact des levures avec une source de carbone et d'azote avant la ségrégation réductionnelle des chromosomes de la première division de méiose afin d'obtenir des levures recombinées ;
d) la récupération des levures recombinées ; et
e) le criblage ou la sélection des levures recombinées afin d'identifier celles présentant l'amélioration recherchée.

De préférence, la souche de levure d'intérêt industriel présente un degré de ploïdie supérieur ou égal à 2.

La présente invention concerne également l'utilisation du processus de RTG de la levure pour améliorer une souche de levure d'intérêt industriel, ledit processus de RTG étant induit par le transfert de la levure d'un milieu riche à un milieu de sporulation, de préférence sans source de carbone fermentescible ni d'azote, l'incubation des levures dans le milieu de sporulation durant un temps suffisant pour induire des cassures double-brin Spo11-dépendantes et la mise en contact des levures avec une source de carbone et d'azote avant la ségrégation réductionnelle des chromosomes de la première division de méiose. De préférence, la souche de levure d'intérêt industriel présente un degré de ploïdie supérieur ou égal à 2.

Le processus de RTG implique que la levure initie un cycle méiotique et des cassures double-brin avant de reprendre un mode de croissance mitotique. L'initiation du cycle méiotique dépend de plusieurs signaux : la présence des deux allèles de type sexuel MATa et MATa, l'absence de source d'azote et de carbone fermentescible.

Tel qu'utilisé dans ce document, le terme « milieu riche » se réfère à un milieu de culture comprenant une source de carbone fermentescible et une source d'azote ainsi que tous les éléments nutritifs nécessaires aux levures pour se multiplier par division mitotique. Ce milieu peut être aisément choisi par l'homme du métier et peut, par exemple, être sélectionné parmi le groupe constitué du milieu YPD (1% extrait de levures, 2% bactopeptone et 2% glucose), du milieu YPG (1% extrait de levures, 2% bactopeptone et 3% glycérol) (Treco and Lundblad, 2001) et d'un milieu synthétique complet (ou milieu SC).

Tel qu'utilisé dans ce document, le terme « milieu de sporulation » se réfère à tout milieu induisant l'entrée en prophase de méiose des cellules de levure sans croissance végétative, en particulier à un milieu de culture ne comprenant pas de source de carbone fermentescible ni de source d'azote mais comprenant une source de carbone métabolisable par respiration telle que l'acétate. Ce milieu peut être aisément choisi par l'homme du métier et peut, par exemple, être sélectionné parmi le groupe constitué du milieu KAc 1% (Wu and Lichten, 1994), le milieu SPM (Kassir and Simchen, 1991) et des milieux de sporulation décrits dans l'article de Sherman (Sherman, 1991).

Selon un mode de réalisation préférée, avant d'être incubées dans le milieu de sporulation, les cellules sont cultivées durant quelques cycles de division dans un milieu de pré-sporulation de façon à obtenir une sporulation efficace et synchrone. Le milieu de pré-sporulation peut être aisément choisi par l'homme du métier. Ce milieu peut être, par exemple, le milieu SPS (Wu and Lichten, 1994).

Le choix des milieux (milieu riche, milieu de pré-sporulation, milieu de sporulation) dépend des caractéristiques physiologiques et génétiques de la souche de levure à améliorer, notamment si cette souche est auxotrophe pour un ou plusieurs composés.

La réversibilité de la prophase de méiose est transitoire. En effet, si les cellules sont ré-incubées dans un milieu riche avant la formation des cassures double-brin Spo11-dépendantes, celles-ci reprennent rapidement leur division mitotique sans modification du génome. A l'inverse, si les cellules sont remises en culture dans un milieu riche après un stade d'engagement qui se situe juste avant la ségrégation réductionnelle des chromosomes lors de la première division méiotique (MI), la sporulation se poursuit normalement et génère des spores recombinées. Ainsi, le processus de RTG, créant des génotypes recombinés, peut se produire dans une fenêtre de temps se situant entre la formation des cassures double-brin Spo 11-dépendantes et le stade d'engagement avant la ségrégation réductionnelle des chromosomes en MI.

La durée d'incubation dans le milieu de sporulation peut varier selon les souches de levures. En effet, pour certaines souches répondant plus lentement aux signaux de carence nutritionnelle, la durée d'incubation dans ce milieu peut être augmentée.

La durée d'incubation dans le milieu de sporulation peut être déterminée par l'homme du métier pour chaque souche d'intérêt en déterminant (i) la période de formation des cassures double-brin Spo 11-dépendantes, (ii) la période de ségrégation réductionnelle des chromosomes et (iii) le stade d'engagement avant la ségrégation réductionnelle des chromosomes. Alternativement, la durée d'incubation dans le milieu de sporulation peut être déterminée par l'homme du métier pour chaque souche d'intérêt en déterminant uniquement le stade d'engagement. L'ajout d'une source de carbone et d'azote peut, par exemple, être envisagé 1, 2, 3 ou 4 heures, de préférence 1 ou 2 heures, avant le stade d'engagement.

La formation des cassures double-brin Spo 11-dépendantes peut être suivie par des techniques classiques de biologie moléculaire telles que le Southern-blot. De la même manière, il est possible d'observer la ségrégation réductionnelle des chromosomes par simple marquage de l'ADN, par exemple par marquage DAPI. Le stade d'engagement avant lequel les cellules doivent être remises en présence d'une source de carbone et d'azote, se situe juste avant la ségrégation réductionnelle des chromosomes. Ce stade peut être déterminé en menant des expériences de RTG au cours desquelles les cellules sont remises en présence d'une source de carbone et d'azote à différents temps d'incubation. Il est ainsi possible de définir la fenêtre de temps durant laquelle le pourcentage de cellules reprenant une division mitotique (que l'on peut visualiser par la naissance d'un bourgeon cellulaire) est le plus important (et par conséquent durant laquelle le pourcentage de cellules poursuivant le processus de sporulation est le plus faible). A titre d'exemple, pour les levures *Saccharomyces cerevisiae* de fond génétique S288C ou SK1 telles qu'utilisées dans la partie expérimentale, cette fenêtre de temps est de 5 à 7 heures après le transfert des cellules dans le milieu de sporulation.

Afin d'être remises en présence d'une source de carbone et d'azote, les levures peuvent être transférées dans un milieu riche tel que décrit ci-dessus. De manière alternative, une source de carbone et une source d'azote peuvent être ajoutées directement au milieu de sporulation.

La source de carbone peut être toute source de carbone fermentescible et peut être, par exemple, sélectionnée dans le groupe constitué du glucose, galactose, glycérol, saccharose, fructose et maltose. La source d'azote peut être, par exemple, sélectionnée dans le groupe constitué d'azote inorganique tel que le sulfate d'ammonium, le phosphate d'ammonium, le nitrate de sodium, le nitrate d'ammonium ou le nitrate de potassium, et d'azote organique, par exemple sous forme d'acides aminés (glutamate, glutamine) ou d'extraits de levure (Albers et al., 1996).

Les levures remises en contact avec une source de carbone fermentescible et d'azote reprennent une division mitotique après un temps d'adaptation durant lequel les cassures double-brin sont réparées. Les très nombreux évènements de recombinaison observés par les inventeurs ont lieu durant cette période lors de la réparation de ces cassures. La durée de cette période d'adaptation peut être variable selon les espèces et est aisément ajustable par l'homme du métier. De manière générale, elle peut durer de 1 à 3 heures, de préférence environ 1h30.

Après cette période d'adaptation, les levures recombinées peuvent être récupérées (étape (d) du procédé) pour effectuer un criblage ou une sélection. Optionnellement, avant le criblage ou la sélection, elles peuvent être conservées sous forme de banques de levures recombinées.

La souche de levure d'intérêt industriel peut être haploïde (n chromosomes), diploïde (2n chromosomes) ou polyploïde (2n, 3n, 4n ou plus chromosomes). La souche de levure d'intérêt industriel peut également être aneuploïde, c'est-dire présenter une ploïdie qui n'est pas un multiple exact du nombre haploïde. Dans ce cas, la levure peut comprendre par exemple n+x, 2n+x ou 3n+x chromosomes, où x est le nombre de chromosomes additionnels par rapport au multiple exact du nombre haploïde. La levure peut par exemple présenter une ou plusieurs disomies, c'est-à-dire avoir deux copies d'un ou plusieurs chromosomes (par exemple n+1, n+2 ou n+3 chromosomes) ou une ou plusieurs trisomies c'est-à-dire avoir trois copies d'un ou plusieurs chromosomes (par exemple n+2, 2n+1 ou 2n+2 chromosomes).

Selon un mode de réalisation particulier, la souche de levure d'intérêt industriel comprend au minimum une disomie.

De préférence, la souche de levure d'intérêt industriel présente un degré de ploïdie supérieur ou égal à 2. En particulier, celle-ci peut être diploïde (2n), aneuploïde (par exemple 2n+x ou 3n+x) ou polyploïde (par exemple 3n, 4n ou 5n), de préférence diploïde.

Afin d'être soumise au processus de RTG, la souche de levure d'intérêt est capable d'entrer en sporulation et de progresser jusqu'au stade de formation des cassures double brin. En revanche, elle peut être déficiente pour les étapes ultérieures et incapable de former des spores matures et/ou viables. L'entrée en sporulation dépend notamment d'un signal sexuel composé des deux allèles a et α. Dans la cas où la levure est haploïde ou aneuploïde et ne comprend qu'un seul de ces allèles, il est possible d'induire des mutations conduisant à l'expression des deux types sexuels (par exemple des mutations dans les gènes *sir* qui régulent l'expression des loci sexuels HMR et HML) ou d'introduire dans le génome un gène sexuel portant l'allèle manquant. De telles modifications ont été décrites par exemple chez *Saccharomyces cerevisiae* afin d'induire l'entrée en méiose de souches haploïdes (De Massy et al., 1994).

Selon un mode de réalisation préférée, la souche de levure d'intérêt industriel est une levure hybride, de préférence une levure diploïde hybride. En particulier, la souche de levure d'intérêt industriel peut être obtenue par le croisement d'une souche d'intérêt avec une autre souche génétiquement distincte et présentant une caractéristique d'intérêt. La cellule hybride ainsi obtenue est ensuite transférée dans le milieu de sporulation (étape (a) du procédé). Ainsi, le procédé selon l'invention peut comprendre en outre, avant l'étape a), les étapes de (i) sélection d'une souche de levure génétiquement distincte d'une souche de levure d'intérêt industriel et présentant une caractéristique d'intérêt destinée à améliorer cette dernière, et de (ii) croisement de la souche d'intérêt et de la souche génétiquement distincte afin d'obtenir une souche hybride. Les caractères génétiques des deux souches parentes sont ensuite recombinés à l'aide du procédé selon l'invention.

Le terme « levure hybride » ou « souche hybride » tel qu'utilisé dans ce document, se réfère à une levure obtenue par croisement de deux souches génétiquement distinctes, c'est-à-dire de deux souches présentant une différence au niveau de leur génotype ou d'au moins un trait génétique. De préférence, les souches parentes diffèrent au niveau d'au moins une caractéristique phénotypique telle que, par exemple, la vitesse de croissance, la thermotolérance, la cryotolérance, la sensibilité au pH, la capacité et la rapidité de fermentation, la résistance à l'éthanol ou à tout autre composé présent dans le milieu de fermentation ou excrété par la culture cellulaire, la morphologie cellulaire, le caractère de floculation, la sensibilité à une molécule particulière, l'efficacité de sporulation, les profils aromatiques, les exigences nutritionnelles, la résistance au séchage, ou encore la fermentation d'un sucre particulier. Les levures hybrides peuvent être diploïdes, aneuploïdes, polyploïdes, et en particulier allopolyploïdes, c'est-à-dire comprenant plusieurs jeux de chromosomes provenant d'espèces différentes. De préférence, la levure hybride est diploïde. La nature hybride peut être intra ou inter-espèces, de préférence inter-espèces. Les hybrides inter-espèces sont le plus souvent stériles. Les levures hybrides peuvent être obtenues par des méthodes bien connues de l'homme du métier, notamment par fusion de deux spores ou de deux protoplastes (hybridation somatique).

Les levures issues d'hybridations intra ou inter-espèces sont fréquemment utilisées dans l'industrie. L'hybridation peut en effet être utilisée pour créer de nouvelles souches de levures présentant une combinaison des caractéristiques provenant des souches parentes. Pour l'industrie oenologique, par exemple, il peut être intéressant de croiser une souche présentant une bonne résistance à l'éthanol avec une souche résistant à des températures élevées. Les hybrides présentant la combinaison des caractéristiques d'intérêt des souches parentes sont ensuite sélectionnés.

L'un des principaux inconvénients dans l'utilisation de souches hybrides, provient du fait que ces souches sont fréquemment stériles. La stérilité d'une souche peut notamment provenir de son incapacité à produire des spores matures ou de la non-viabilité des spores. A titre d'exemple, les levures hybrides diploïdes obtenues par la fusion de *Saccharomyces cerevisiae* et *Saccharomyces paradoxus* et utilisées dans la production de vin rouge, sont capables de se multiplier par croissance mitotique mais sont incapables de produire des gamètes haploïdes viables (Greig, 2007). Ces souches hybrides stériles ne peuvent donc pas être modifiées plus avant par croisement entre descendants ou par fusion avec d'autres souches d'intérêt.

En revanche, ces souches peuvent être recombinées selon le procédé de l'invention. Ainsi, selon un mode de réalisation préféré, la souche de levure d'intérêt industriel est une souche hybride stérile, et en particulier une souche hybride diploïde stérile. En effet, les inventeurs ont démontré que le processus de RTG permettait d'obtenir des souches hautement recombinées sans qu'il ne soit nécessaire de compléter le processus de sporulation. Le procédé selon l'invention permet donc d'obtenir des cellules hautement recombinées à partir d'hybrides stériles et ainsi d'améliorer une souche hybride sans pour autant avoir recours à des techniques d'ADN recombinant.

Le terme « levure stérile » tel qu'utilisé dans ce document, se réfère à une levure incapable ou ayant une capacité réduite à produire des spores matures et/ou une levure dont les spores ne sont pas viables ou ont une viabilité réduite. La levure stérile est en revanche capable d'entrer en sporulation et de progresser jusqu'au stade de formation des cassures double brin. La levure stérile peut avoir une capacité à produire des spores matures réduite de 50, 60, 70, 80, 90, 95 ou 99% par rapport à une souche de référence de la même espèce ou, dans le cas d'une levure hybride, par rapport à une des souches parentes. La levure stérile peut également produire des spores ayant une viabilité réduite. En particulier, parmi les spores produites par cette levure, au moins 25, 50 ou 75 % des spores peuvent être non viables. De préférence, la souche stérile est incapable de produire des spores matures ou ne produit pas de spores viables.

La souche de levure d'intérêt industriel peut être destinée, ou être utilisée, dans tout procédé industriel, en particulier dans les procédés de l'agro-alimentaire tels que la vinification, la brasserie, la distillerie ou la panification, dans la production de biocarburants, dans la production de molécules d'intérêt ou encore dans la dégradation de la cellulose.

La souche d'intérêt industriel peut être toute souche de levure qui, lorsqu'elle est remise en présence d'une source de carbone et d'azote, est capable de reprendre une division mitotique après induction de cassures double-brin en prophase de méiose.

Selon un mode de réalisation préféré, la souche de levure d'intérêt industriel est un organisme non génétiquement modifié. Tel qu'utilisé dans ce document, le terme d'« organisme non génétiquement modifié » se réfère à des levures dont le patrimoine génétique a été modifié par génie génétique, en particulier par transgénèse.

De préférence, les levures recombinées obtenues par le procédé selon l'invention ne sont pas considérées comme des organismes génétiquement modifiés (OGM).

Selon un mode de réalisation particulier, la souche de levure d'intérêt industriel appartient au genre *Saccharomyces sensu stricto* ou est un hybride obtenu à partir d'une souche appartenant au genre *Saccharomyces sensu stricto*. De préférence, la souche de levure d'intérêt industriel appartient à une espèce sélectionnée dans le groupe constitué de *Saccharomyces cerevisiae, Saccharomyces bayanus, Saccharomyces castelli, Saccharomyces eubayanus, Saccharomyces kluyveri*, *Saccharomyces kudriavzevii, Saccharomyces mikatae*, *Saccharomyces uvarum*, *Saccharomyces paradoxus* and *Saccharomyces pastorianus* (aussi nommée *Saccharomyces carlsbergensis*) ou est un hybride obtenu à partir d'une souche appartenant à l'une de ces espèces. De manière tout particulièrement préférée, la souche de levure d'intérêt industriel appartient à l'espèce *Saccharomyces cerevisiae* ou est un hybride obtenu à partir d'une souche appartenant à l'espèce *Saccharomyces cerevisiae*, tel que par exemple, un hybride *S*. *cerevisiae*/ *S*. *paradoxus* ou un hybride *S*. *cerevisiae*/*S*. *uvarum* (Rainieri et al., 1999).

Selon un mode de réalisation, les cellules recombinées obtenues après le processus de RTG (à l'étape (d) du procédé selon l'invention) sont hautement recombinées et présentent plusieurs évènements de recombinaison par cellule, de préférence plus de 2, 3, 5, 10, 15, 20, 25, 30, 35, 40, 45 ou 50 événements de recombinaison par cellule. Chaque événement peut se traduire par un crossing-over, une conversion génique ou une conversion génique associée à un crossing-over selon le mécanisme impliqué dans la réparation de la cassure double-brin. Tel qu'utilisé dans ce document, le terme « événement de recombinaison » se réfère à une jonction recombinante observée dans le génotype d'une cellule issue du RTG (i) entre une région mono-allélique, de préférence d'au minimum 20 kb, et une région bi-allélique ou (ii) entre deux régions mono-alléliques d'allèles différents, de préférence une de ces régions ayant une taille d'au minimum 20 kb.

Selon un mode de réalisation particulier, les événements de recombinaison induisent une diminution du taux d'hétérozygotie. Cela signifie que des événements de recombinaison se traduisent par des pertes d'hétérozygotie. La création de régions homozygotes est particulièrement intéressante dans les procédés d'amélioration de souches étant donné qu'elle permet l'expression de traits récessifs potentiellement intéressants. La diminution du taux d'hétérozygotie peut être évaluée, par exemple, par le suivi de marqueurs de polymorphisme génétique, en particulier de polymorphisme nucléotidique.

Un autre avantage du procédé selon l'invention est que les cellules recombinées peuvent avoir un degré de ploïdie supérieur ou égal à 2, de préférence égal à 2. De ce fait, les caractères quantitatifs s'exprimant à l'état hétérozygote (phénomène d'hétérosis) peuvent être facilement analysés et les cellules recombinées peuvent être directement utilisées à leur tour dans le procédé selon l'invention pour subir d'autres recombinaisons.

Dans un mode de réalisation, les levures recombinées sont à nouveau transférées dans un milieu de sporulation, de préférence sans source de carbone fermentescible ni d'azote, afin d'induire l'entrée des levures en prophase de méiose, sont incubées dans le milieu de sporulation durant un temps suffisant pour induire des cassures double-brin Spo11-dépendantes et sont mises en contact avec une source de carbone et d'azote avant la ségrégation réductionnelle des chromosomes de la première division de méiose avant d'être récupérées. Le criblage ou la sélection des levures recombinées peut avoir lieu à la fin de chaque cycle ou après plusieurs cycles. Lorsque le criblage ou la sélection des levures recombinées a lieu à la fin de chaque cycle, seules les levures sélectionnées peuvent être à nouveau soumises au procédé d'amélioration. Les étapes a) à d) ou a) à e) du procédé selon l'invention peuvent ainsi être répétées une ou plusieurs fois. En particulier, les étapes a) à d) ou a) à e) du procédé selon l'invention peuvent être répétées au moins 1, 2, 3, 4 ou 5 fois. De préférence, les étapes a) à d) du procédé selon l'invention peuvent être répétées au moins 1, 2, 3, 4 ou 5 fois, avant de procéder au criblage ou à la sélection des levures recombinées présentant l'amélioration recherchée.

De façon optionnelle, les levures recombinées récupérées à l'étape d) ou sélectionnées à l'étape e) peuvent être induites en sporulation pour obtenir des spores haploïdes. Les spores d'une tétrade obtenues à partir d'une cellule recombinée peuvent être séquencées afin de déterminer l'haplotype des chromosomes de ladite cellule recombinée. De manière alternative, les levures recombinées récupérées à l'étape d) ou sélectionnées à l'étape e) peuvent être laissées bourgeonner pour obtenir des couples de cellules « mère/fille ». Ces cellules mère/fille issues d'une cellule recombinée peuvent être séquencées afin de déterminer l'haplotype des chromosomes de ladite cellule recombinée.

Le procédé selon l'invention peut en outre comprendre d'autres étapes permettant d'augmenter ou de cibler les événements de recombinaison dans les levures lors du processus de RTG. Selon un mode de réalisation, lorsque les levures sont dans le milieu de sporulation (étape a) du procédé), celles-ci sont exposées à un ou plusieurs agents mutagènes chimiques tels que du méthyle méthane sulfonate (MMS), ou physiques tels que des ultra-violets ou des radiations ionisantes. Cette exposition permet ainsi d'opérer une double diversification génétique par recombinaison des marqueurs polymorphiques et mutagénèse aléatoire.

Selon un autre mode de réalisation, une construction d'acide nucléique telle que décrite dans le brevet européen EP 1523564 est introduite dans la levure à améliorer avant que celle-ci ne soit transférée dans le milieu de sporulation. Cet acide nucléique code pour une protéine de fusion, sous le contrôle d'un promoteur spécifique ou non spécifique, de préférence spécifique, de la méiose, comprenant un domaine de liaison à l'ADN (par exemple Gal4BD) lié de façon opérationnelle à une protéine Spo11 et permet d'augmenter la fréquence des cassures double-brin sur certaines régions chromosomiques en prophase de méiose ou de modifier la distribution desdites cassures sur l'ensemble du génome. Alternativement, la protéine de fusion comporte, sous le contrôle d'un promoteur spécifique de la méiose, un domaine de liaison à l'ADN lié de façon opérationnelle à une protéine partenaire de Spo11, impliquée dans la formation de cassures doubles brins et capables de recruter Spo11. La protéine partenaire de Spo11 peut être notamment choisie parmi celles décrites dans l'article de Keeney (2001), Smith et Nicolas (1998), Acquaviva et al., (2012). En particulier, elle peut être sélectionnée dans le groupe constitué de ME14, MER2/REC107, REC102, REC104, REC114, REC103/SK18, MRE2/NAM8, MRE11, RAD50, XRS2/NBS1, HOP1, RED1, MER1, MEK1, SET1 et SPP1.

Le criblage ou la sélection des levures recombinées présentant l'amélioration recherchée peut être effectué par toute méthode connue de l'homme du métier et dépend de la caractéristique recherchée. Du fait de la très grande variété des applications industrielles des levures, et en particulier des levures appartenant à l'espèce *Saccharomyces cerevisiae*, de nombreuses caractéristiques peuvent être améliorées à l'aide du procédé selon l'invention. Ces caractéristiques peuvent être, par exemple, la vitesse de croissance, en particulier dans des conditions particulières, la résistance aux températures élevées (thermotolérance) ou au contraire aux basses températures (cryotolérance), la sensibilité au pH, la capacité et la rapidité de fermentation, la résistance à l'éthanol ou à tout autre composé présent dans le milieu de fermentation ou excrété par la culture cellulaire, la morphologie cellulaire, le caractère de floculation, la sensibilité à une molécule particulière, l'efficacité de sporulation, les profils aromatiques, les exigences nutritionnelles, la résistance au séchage, ou encore la fermentation d'un sucre particulier.

La présente invention concerne également une levure recombinée obtenue selon le procédé d'amélioration selon l'invention, ou une levure dérivée de celle-ci.

Les inventeurs ont démontré par séquençage complet du génome des cellules issues du processus de RTG, que celui-ci permettait de générer une très grande diversité génétique et qu'aucune des cellules issues de ce processus ne présentait le même profil de recombinaison.

Ainsi, selon un autre aspect, la présente invention concerne l'utilisation du processus de RTG de la levure pour générer une banque de levures recombinées à partir d'une levure, ledit processus RTG étant induit par le transfert de ladite levure d'un milieu riche à un milieu de sporulation, de préférence sans source de carbone fermentescible ni d'azote, l'incubation dans le milieu de sporulation durant un temps suffisant pour induire des cassures double-brin Spo11-dépendantes, et la mise en contact des levures avec une source de carbone et d'azote avant la ségrégation réductionnelle des chromosomes de la première division de méiose.

De préférence, la levure présente un degré de ploïdie supérieur ou égal à 2, et de manière plus particulièrement préférée est diploïde. De préférence, ladite levure est une souche hybride, en particulier une souche hybride stérile.

Elle concerne également un procédé pour générer une banque de levures recombinées à partir d'une levure, de préférence une levure présentant un degré de ploïdie supérieur ou égal à 2, comprenant :
a) le transfert de la levure d'un milieu riche à un milieu de sporulation, de préférence sans source de carbone fermentescible ni d'azote ;
b) l'incubation des levures dans le milieu de sporulation durant un temps suffisant pour induire des cassures double-brin Spo 11-dépendantes :
(c) la mise en contact des levures avec une source de carbone et d'azote avant la ségrégation réductionnelle des chromosomes de la première division de méiose afin d'obtenir des levures recombinées ;
d) la récupération des levures recombinées afin de former une banque de levures recombinées.

Les modes de réalisation décrits ci-dessus pour le processus d'amélioration selon l'invention s'appliquent également à cet aspect.

Selon un mode préféré de réalisation, les levures diploïdes recombinées présentent plusieurs évènements de recombinaison par cellule, de préférence plus de 2, 3, 5, 10, 15, 20, 25, 30, 35, 40, 45 ou 50 évènements de recombinaison par cellule.

Selon un mode de réalisation, le procédé comprend en outre, avant l'étape a), l'étape consistant à obtenir la levure présentant de préférence un degré de ploïdie supérieur ou égal à 2, par croisement de deux levures d'intérêt.

De préférence, la levure présentant un degré de ploïdie supérieur ou égal à 2 est diploïde. De préférence, ladite levure est une souche hybride, en particulier une souche hybride stérile. Selon un mode de réalisation préférée, la levure est une souche d'intérêt industriel, en particulier une souche diploïde d'intérêt industriel. De manière plus particulièrement préférée, la levure appartient au genre *Saccharomyces sensu stricto,* et plus particulièrement à l'espèce *S. cevevisiae*, ou est un hybride obtenu à partir d'une souche appartenant au genre *Saccharomyces sensu stricto,* et plus particulièrement à l'espèce *S*. *cevevisiae*.

La banque de levure ainsi générée peut notamment être utilisée pour sélectionner des souches de levure présentant des caractéristiques particulières ou encore pour localiser un caractère génétique d'intérêt, en particulier un caractère quantitatif d'intérêt (ou QTL), par comparaison des génotypes et phénotypes de levures recombinées.

La présente invention concerne également une banque de levures recombinées obtenue selon le procédé pour générer une banque de levures selon l'invention.

La présente invention concerne également un procédé pour identifier ou localiser l'information génétique codant pour une caractéristique d'intérêt dans une levure, de préférence une levure présentant un degré de ploïdie supérieur ou égal à 2, comprenant :
a) le transfert de la levure d'un milieu riche à un milieu de sporulation, de préférence sans source de carbone fermentescible ni d'azote ;
b) l'incubation des levures dans le milieu de sporulation durant un temps suffisant pour induire des cassures double-brin Spo 11-dépendantes ;
c) la mise en contact des levures avec une source de carbone et d'azote avant la ségrégation réductionnelle des chromosomes de la première division de méiose afin d'obtenir des levures recombinées ;
d) la récupération des levures recombinées ; et
e) l'analyse des génotypes et phénotypes des levures recombinées afin identifier ou localiser l'information génétique codant pour la caractéristique d'intérêt.

Elle concerne aussi l'utilisation du processus de RTG de la levure pour identifier ou localiser l'information génétique codant pour une caractéristique d'intérêt dans une levure, de préférence une levure présentant un degré de ploïdie supérieur ou égal à 2, ledit processus de RTG étant induit par le transfert de la levure d'un milieu riche à un milieu de sporulation, de préférence sans source de carbone fermentescible ni d'azote, l'incubation des levures dans le milieu de sporulation durant un temps suffisant pour induire des cassures double-brin Spo11-dépendantes, et la mise en contact des levures avec une source de carbone et d'azote avant la ségrégation réductionnelle des chromosomes de la première division de méiose.

De manière préférée, la caractéristique d'intérêt est un caractère quantitatif d'intérêt (ou QTL).

Les modes de réalisation décrits ci-dessus pour le processus d'amélioration selon l'invention s'appliquent également à cet aspect.

Les inventeurs ont également montré que sur les 63 901 marqueurs de polymorphisme nucléotidique (SNPs) analysés, en moyenne 27% d'entre eux présentaient une perte d'hétérozygotie dans les cellules issues du RTG. Cette observation est d'autant plus surprenante qu'elle va à l'encontre des enseignements de l'art antérieur qui indiquaient que les mécanismes de réparation des cassures double-brin mis en place lors du RTG semblaient minimiser la résolution des cassures par des phénomènes de crossing-over afin de préserver l'intégrité du génome et de limiter les pertes d'hétérozygotie.

Ainsi, selon encore un autre aspect, la présente invention concerne l'utilisation du processus de RTG pour diminuer le taux d'hétérozygotie d'une levure, de préférence une levure présentant un degré de ploïdie supérieur ou égal à 2, en particulier d'une levure diploïde hybride, ledit processus RTG étant induit par le transfert de la levure d'un milieu riche à un milieu de sporulation, de préférence sans source de carbone fermentescible ni d'azote, l'incubation des levures dans le milieu de sporulation durant un temps suffisant pour induire des cassures double-brin Spo11-dépendantes, et la mise en contact des levures avec une source de carbone et d'azote avant la ségrégation réductionnelle des chromosomes de la première division de méiose.

Elle concerne aussi un procédé pour diminuer le taux d'hétérozygotie d'une levure, de préférence une levure présentant un degré de ploïdie supérieur ou égal à 2, en particulier d'une levure diploïde hybride, comprenant :
a) le transfert de la levure d'un milieu riche à un milieu de sporulation, de préférence sans source de carbone fermentescible ni d'azote ;
b) l'incubation des levures dans le milieu de sporulation durant un temps suffisant pour induire des cassures double-brin Spo11-dépendantes ;
c) la mise en contact des levures avec une source de carbone et d'azote avant la ségrégation réductionnelle des chromosomes de la première division de méiose afin d'obtenir des levures recombinées d) la récupération des levures recombinées, lesdites levures recombinées présentant un taux d'hétérozygotie inférieur à celui de la levure de l'étape a).

Les modes de réalisation décrits ci-dessus pour le processus d'amélioration selon l'invention s'appliquent également à cet aspect.

De préférence, la levure présentant un degré de ploïdie supérieur ou égal à 2 est diploïde. De préférence, ladite levure est une souche hybride, en particulier une souche hybride stérile, de préférence d'intérêt industriel. De manière plus particulièrement préférée, la levure appartient au genre *Saccharomyces sensu stricto,* et plus particulièrement à l'espèce *S. cevevisiae*, ou est un hybride obtenu à partir d'une souche appartenant au genre *Saccharomyces sensu stricto,* et plus particulièrement à l'espèce *S*. *cevevisiae*.

Les exemples qui suivent sont présentés dans un but illustratif et non limitatif.

### EXEMPLES

### Matériel et méthodes :

### Souches de levure

Les informations concernant les souches utilisées, leur origine et leur génotype, sont indiquées dans la Tableau 1 ci-dessous.

**Tableau 1 : Informations concernant les souches utilisées**

| Nom | Origine | Fond génétique | Génotype |
|---|---|---|---|
| ORT3805 | A. Nicolas | S288C | MATa, his1 |
| ORT3806 | A. Nicolas | S288C | MATα, his1 |
| Y00981 | (a) | S288C | MATa, his3Δ1, leu2Δ0, met15Δ0, ura3Δ0, arg4Δ::KanMX |
| ORT7204 | Y00981 | S288C | MATa, his3Δ1, leu2Δ0, met15Δ0, ura3Δ0, arg4Δ::URA3 |
| ORT7219 | (b) | S288C | MATα, ho, lys2Δ0, ura3Δ0, leu2Δ0, his3Δ200, met15Δ0, trp1Δ63 |
| ORT7205 | ORT7219 | S288C | MATα, ho, lys2Δ0, ura3Δ0, arg4Δ::URA3, leu2Δ0, his3Δ200, met15Δ0, trp1Δ63 |
| ORT7235 | ORT7205 | S288C | MATα, ho, lys2Δ0, ura3Δ0, arg4-Bgl, leu2Δ0, his3Δ200, met15Δ0, trp1Δ63 |
| ORT7221 | (c) | SK1 | MATa, ho, lys2, ura3(PstI-SmaI)::hisG, leu2::hisG, his4B::LEU2, trp1::hisG |
| ORT7217 | ORT7221 | SK1 | MATa, ho, lys2, ura3(PstI-SmaI)::hisG, arg4::URA3, leu2::hisG, his4B::LEU2, trp1::hisG |
| ORT7236 | ORT7217 | SK1 | MATa, ho, lys2, ura3(PstI-SmaI)::hisG, arg4-RV, leu2::hisG, his4B::LEU2, trp1::hisG |
| AND1702 | ORT7235 x ORT7236 | Hybride S288C-SK1 | MATa/MATα, ho/ho, arg4-Bgl/arg4-RV, lys2Δ0/lys2, ura3(PstI-SmaI::hisG/ura3Δ0, leu2Δ0/leu2::hisG, his3Δ200/HIS3, met15Δ0/MET15, trp1Δ63/trp1::hisG, his4B::LEU2/HIS4 |
| AND1708 | AND1702 | Hybride S288C-SK1 | MATa/MATα, ho/ho, ARG4/arg4-Bgl, lys2Δ0/lys2Δ0, ura3Δ0/ura3(PstI-SmaI::hisG, trp1Δ63/trpl::hisG ; , leu2Δ0/leu2::hisG his4B::LEU2/his4B::LEU2, his3Δ200/his3Δ200, MET15/met15Δ0 |
| AND1709 | AND1702 | Hybride S288C-SK1 | MATa/MATα, ho/ho, lys2Δ0/lys2Δ0, ura3Δ0/ura3(recombinantΔ0-(PstI-SmaI): :hisG), trp1Δ63/trp1::hisG, leu2Δ0/leu2::hisG, HIS4/his4B::LEU2, his3Δ200/his3Δ200 |
| AND1710 | AND1702 | Hybride S288C-SK1 | MATa/MATα, ho/ho, lys2Δ0/lys2Δ0, ura3Δ0/ura3(recombinant Δ0-(PstI-SmaI::hisG), trp1Δ63/trp1::hisG, leu2Δ0/leu2::hisG, HIS4/his4B::LEU2, his3Δ200/his3Δ200, |
| AND1711 | AND1702 | Hybride S288C-SK1 | MATa/MATα, ho/ho, ARG4/arg4-RV, lys2Δ0/lys2Δ0, ura3Δ0/ura3(PstI-SmaI::hisG, trp1Δ63/trp1::hisG, leu2Δ0/leu2::hisG, HIS4/his4B::LEU2, his3Δ200/his3Δ200 |
| AND1712 | AND1702 | Hybride S288C-SK1 | MATa/MATα, ho/ho, ARG4/arg4-RV, lys2Δ0/lys2, ura3Δ0/ura3(PstI-SmaI::hisG, trp1Δ63/trp1::hisG, leu2Δ0/leu2::hisG, HIS4/his4B::LEU2, his3Δ200/his3Δ200, MET15/met15Δ0 |
| AND1720 | AND1702 | Hybride S288C-SK1 | MATa/MATα, ho/ho, ARG4/arg4-Bgl, lys2Δ0/lys2, ura3Δ0/ura3(PstI-SmaI::hisG, trp1Δ63/trp1::hisG, leu2Δ0/leu2::hisG, HIS4/his4B::LEU2, his3Δ200/HIS3, met15Δ0/met15Δ0 |
| AND1733(M) | AND1702 | Hybride S288C-SK1 | MATa/MATα, ho/ho, ARG4/arg4-Bgl, lys2Δ0/lys2 ; ura3Δ0/ura3(PstI-SmaI)::hisG, trp1Δ63/trp1::hisG, leu2::hisG/leu2::hisG, his4B::LEU2/his4B::LEU2, MET15/met15Δ0 |
| AND1734(F) | AND1702 | Hybride S288C-SK1 | MATa/MATα, ho/ho, arg4-Bgl/arg4-RV, lys2Δ0/lys2, ura3Δ0/ura3(PstI-SmaI::hisG, trp1Δ63/trp1::hisG, leu2Δ0/leu2Δ0, MET15/met15Δ0 |
| AND1735(M) | AND1702 | Hybride S288C-SK1 | MATa/MATα, ho/ho, arg4-Bgl/arg4-RV, lys2Δ0/lys2, ura3Δ0/ura3(PstI-SmaI)::hisG, trp1Δ63/trp1::hisG, leu2Δ0/leu2::hisG ;his3Δ200/his3Δ200, his4B::LEU2/HIS4, MET15/met15Δ0 |
| AND1736(F) | AND1702 | Hybride S288C-SK1 | MATa/MATα, ho/ho, arg4-Bgl/arg4-RV, lys2Δ0/lys2, ura3Δ0/ura3(PstI-SmaI::hisG, trp1Δ63/trp1::hisG, leu2Δ0/leu2::hisG, his4B::LEU2/HIS4, MET15/met15Δ0 |
| AND1737(M) | AND1702 | Hybride S288C-SK1 | MATa/MATα, ho/ho, arg4-Bgl/arg4-RV, lys2Δ0/lys2, ura3Δ0/ura3(PstI-SmaI::hisG, trp1Δ63/trp1::hisG, leu2Δ0/leu2Δ0, HIS3/his3Δ200, , MET15/met15Δ0 |
| AND1738(F) | AND1702 | Hybride S288C-SK1 | MATa/MATα, ho/ho, arg4-Bgl/arg4-RV, lys2Δ0/lys2, ura3Δ0/ura3(PstI-SmaI)::hisG, trp1Δ63/trp1::hisG, leu2::hisG/leu2::hisG, his3Δ200/HIS3, his4B::LEU2/his4B::LEU2, MIET15/met15Δ0 |
| AND1739(M) | AND1702 | Hybride S288C-SK1 | MATa/MATα, ho/ho, arg4-Bgl/arg4-RV, lys2Δ0/lys2, ura3Δ0/ura3(PstI-SmaI::hisG, trp1Δ63/trp1::hisG, leu2Δ0/leu2::hisG ; HIS4/his4B::LEU2 , MET15/met15Δ0 |
| AND1740(F) | AND1702 | Hybride S288C-SK1 | MATa/MATα, ho/ho, arg4-Bgl/arg4-RV, lys2Δ0/lys2, ura3Δ0/ura3(PstI-SmaI)::hisG, trp1Δ63/trp1::hisG, leu2Δ0/leu2::hisG, his3Δ200/his3Δ200, HIS4/his4B::LEU2, MET15/met15Δ0 |
| AND1710-1A | AND1710 | Hybride S288C-SK1 | MATα, ho, ARG4, lys2Δ0, ura3Δ0, trp1::hisG, leu2::hisG, his3Δ200, |
| AND1710-1B | AND1710 | Hybride S288C-SK1 | MATa, ho, ARG4, lys2Δ0, ura3(recombinant Δ0-(PstI-SmaI)::hisG), trp1Δ63, leu2Δ0, his3Δ200, his4B::LEU2 |
| AND1710-1C | AND1710 | Hybride S288C-SK1 | MATa, ho, ARG4, lys2Δ0, ura3Δ0, trp1::hisG, leu2::hisG, his3Δ200, his4B::LEU2 |
| AND1710-1D | AND1710 | Hybride S288C-SK1 | MATα, ho, ARG4, lys2Δ0, ura3(recombinant Δ0-(PstI-SmaI)::hisG), trp1Δ63, leu2Δ0, his3Δ200 |
| AND2711 | AND1735 | Hybride S288C-SK1 | MATα/MATa, ho/ho, arg4-Bgl/arg4-RV, lys2/lys2, ura3Δ0/ura3(PstI-SmaI::hisG, leu2Δ0/leu2::hisG, trp1Δ63/trp1::hisG, his3Δ200/his3Δ200, HIS4/his4B::LEU2 |
| AND2907 | AND2711 | Hybride S288C-SK1 | MATa/MATa, ho/ho, arg4-Bgl/arg4-RV, lys2/lys2, ura3Δ0/ura3(PstI-SmaI::hisG, leu2Δ0/leu2::hisG, trp1Δ63/trp1::hisG, his3Δ200/his3Δ200, HIS4/his4B::LEU2 |
| ORT7469 | ORT7235 | S288C- | MATα, ho, lys2Δ0, ura3Δ0, arg4-Bgl, leu2Δ0, his3Δ200, met15Δ0, trp1Δ63, ndt80::KanMX |
| ORT7477 | ORT7237 | SK1 | MATa, ho, lys2, ura3(PstI-SmaI)::hisG, arg4-RV, leu2::hisG, his4B::LEU2, trp1::hisG, ndt80::KanMX |
| AND2248 | ORT7469X ORT7477 | Hybride S288C-SK1 | MATa/MATα, ho/ho, lys2/lys2Δ0, ura3(PstI-SmaI)::hisG/ura3Δ0,arg4-RV/arg4-BgI, leu2::hisG/leu2Δ0, HIS3/his3Δ200, his4B::LEU2/HIS4,MET15/met15A0, trp1::hisG/trp1Δ63, ndt80::KanMX/ndt80::KanMX |
| AND2642 | AND2248 | Hybride S288C-SK1 | MATα/MATa, ho/ho, arg4-Bg1/arg4-RV, lys2/lys2, ura3Δ0/ura3(PstI-SmaI::hisG, leu2Δ0/leu2::hisG, his3Δ200/HIS3, met15Δ0/met15Δ0, trp1Δ63/trp1::hisG, HIS4/his4B::LEU2, ndt80Δ::kanMX/ndt80Δ::kanMX |
| AND2652 | AND2248 | Hybride S288C-SK1 | MATα/MATa ; ho/ho ; arg4-Bgl/arg4-RV ; lys2Δ0/lys2 ; ura3Δ0/ura3(PstI-SmaI::hisG ; leu2Δ0/leu2::hisG ; his3Δ200/HIS3 ; met15Δ0/MET15 ; trp1Δ63/trp1::hisG ; his4B::LEU2/his4B::LEU2 ; ndt80Δ::kanMX/ndt80Δ::kanMX |
| AND2658 | AND2248 | Hybride S288C-SK1 | MATα/MATa ; ho/ho ; arg4-Bg1,RV/arg4-RV ; lys2Δ0/lys2 ; ura3Δ0/ura3(PstI-SmaI::hisG ; leu2Δ0/leu2::hisG ; his3Δ200/his3Δ200 ; met15Δ0/MET15 ; trp1Δ63/trp1::hisG ; HIS4/his4B::LEU2 ; ndt80Δ::kanMX/ndt80Δ::kanMX |

| | | | |
|---|---|---|---|
| (a) EUROSCARF (http://web.uni-frankfurt.de/fb15/mikro/euroscarf/data/YHR018c.html) (b) Ben-Ari et al., 2006, souche FY1338 (c) Kane and Roth, 1974, souche dérivée de SK-1 (M) cellule mère (F) cellule fille | | | |

Il s'agit de souches de levure du genre *Saccharomyces cerevisiae* de fond génétique S288C (Mortimer and Johnston, 1986) ou SK1 (Kane and Roth, 1974). Les souches ORT7235 et ORT7236 porteuses respectivement des mutations arg4-Bgl et arg4-RV ont été obtenues en deux étapes de transformation.

Préalablement, la souche Y00981 de la collection de délétions EUROSCARF (http://web.uni-frankfurt.de/fb15/mikro/euroscarf/data/YHR018c.html), où le gène ARG4 est remplacé par KanMX a été transformée par la méthode d'électroporation (Becker and Guarente, 1991) avec le plasmide M4758 (Voth et al., 2003) afin de remplacer KanMX par le gène URA3 au locus ARG4. Les amorces RL1 et RL2 (décrits dans le Tableau 2) ont été utilisés pour amplifier le fragment arg4Δ::URA3.

**Tableau 2 : description des amorces utilisées pour amplifier le locus ARG4**

| Nom | Description | Séquence | Coordonnées |
|---|---|---|---|
| RL1 | fwd-arg4-220 | 3'-TACTCATTGGCAGAATCCCG-5' (SEQ ID NO. : 1) | Chr. VIII : 141604-141623 |
| RL2 | rev-arg4-240 | 3'-CGCTTGAGAGGAAGATTAGC-5' (SEQ ID NO. : 2) | Chr. VIII : 139771-139790 |

Pour obtenir la souche ORT7235, la souche ORT7219 [ARG+ ura-] a été transformée par la méthode d'électroporation en intégrant le produit de PCR arg4Δ::URA3 au locus ARG4. Le transformant obtenu, ORT7205 [arg- URA+], sélectionné sur milieu DO-Uracile, a ensuite été transformé par la méthode d'électroporation avec le fragment arg4-Bgl, obtenu par digestion du plasmide pMY232 (Rocco et al., 1992) par l'enzyme de restriction *PstI*. Le transformant obtenu, ORT7235 [arg- ura-] a été sélectionné sur milieu 5-FOA.

Pour obtenir la souche ORT7236, la souche ORT7221 [ARG+ ura-] a été transformée par la méthode d'électroporation en intégrant le produit de PCR arg4Δ::URA3 au locus ARG4. Le transformant obtenu, ORT7217 [arg- URA+], sélectionné sur milieu DO-Uracile, a ensuite été transformé avec la méthode lithium-acétate (Schiest1 and Gietz, 1989) avec le fragment arg4-RV, obtenu par digestion du plasmide pNPS308 (Rocco et al., 1992) par l'enzyme de restriction PstI. Le transformant obtenu, ORT7236 [arg- ura-] a été sélectionné sur milieu 5-FOA.

La souche diploïde AND1702 a été obtenue par croisement des souches haploïdes ORT7235 et ORT7236. Les deux souches parentales, respectivement de génotype bis3Δ200 et his4::LEU2, sont auxotrophes pour l'histidine, mais le diploïde résultant est prototrophe pour l'histidine par complémentation fonctionnelle et a donc pu être sélectionné sur milieu DO-Histidine. Ainsi, la souche AND1702 est constituée d'un fond génétique hybride S288C-SK1 et est hétérozygote pour les marqueurs génétiques MATa/MATα, arg4-Bg1/arg4-RV, lys2Δ0/lys2, ura3(PstI-SmaI)::hisG/ura3Δ0, leu2Δ0/leu2::hisG, his3Δ200/HIS3, met15Δ0/MET15, trp1Δ63/trp1::hisG, his4B::LEU2/HIS4, et pour tous les marqueurs de polymorphisme qui différencient le génome des deux souches parentales.

Pour obtenir la souche AND2248, les souches ORT7235 et ORT7236 ont été transformées par la méthode d'électroporation en intégrant le produit de PCR *ndt80Δ::KanMX* au locus NDT80. Les deux souches résultantes ORT7469 et ORT7477 ont été croisées pour créer la souche diploïde AND2248.

### Composition des milieux de culture utilisés

Le milieu de croissance YPD est un milieu riche composé de : 1% extraits de levure, 2% bacto-peptone, 2% glucose, (2% bacto-agar si milieux solide), pH 5.5, H₂O ajusté à 1 litre (Treco and Lundblad, 2001). Le milieu YPD contenant de l'arsénite de sodium (NaASO2) est supplémenté avec une concentration finale de 1,5mM.

Le milieu de croissance YPG est un milieu de sélection des cellules capables de respiration composé de : 1% extraits de levure, 2% bactopeptone, 3% glycérol, 2% bacto-agar, H₂O ajusté à 1 litre (Treco and Lundblad, 2001). Le milieu YPG+généticine (200mg/l) permet la sélection des souches résistantes à la généticine, résultant de l'expression du gène KanMX.

Les milieux « drop-out » -X, ou DO -X, sont des milieux synthétiques complets dépourvus d'un nutriment (X), tous les autres étant présents (par exemple DO-Arginine, tous les nutriments sont présents sauf l'arginine). Ils permettent la sélection de souches prototrophes pour le nutriment X et sont composés de : 0,17% base azotée de levure sans acide aminé et sans sulfate d'ammonium, 0,5% sulfate d'ammonium, 2% glucose, 2% bacto-agar, supplémenté en nutriments sauf un, H₂0 ajusté à 1 litre. Les nutriments sont ajoutés dans les proportions suivantes : 0,002% arginine / histidine / méthionine / uracile, 0,003% lysine / tyrosine, 0,004% adénine / tryptophane, 0,005% phénylalanine, 0,006% leucine, 0,01% acide aspartique / acide glutamique, 0,015% valine, 0,02% thréonine, 0,0375% sérine. Les acides aminés non listés peuvent être ajoutés à 0,004% (Treco and Lundblad, 2001). Le milieu de croissance synthétique complet ou SC est équivalent au milieu drop-out sans omission de nutriment.

Le milieu DOBA est un milieu minimum, sur lequel seules les cellules prototrophes peuvent pousser. Il est composé de 0,17% base azotée de levure sans acide aminé et sans sulfate d'ammonium, 0,5% sulfate d'ammonium, 2% glucose, 2% bacto-agar, H₂0 ajusté à 1 litre (Treco and Lundblad, 2001). Il permet la sélection de diploïdes issus du croisement de n'importe quelle souche HIS1 avec l'une ou l'autre des souches de test MATa his1 (ORT3805) ou MATa his1 (ORT3806) par complémentation fonctionnelle.

Le milieu 5-FOA est un milieu de sélection des souches ura3- composé de 2% DO-TRP, 0,17% base azotée de levure sans acide aminé et sans sulfate d'ammonium, 0,5% sulfate d'ammonium, 0,00204% tryptophane, 0,003% uracile, 0,15% acide 5-fluoro-orotique, 2% glucose, 2% bacto-agar, pH 4.5, H₂0 ajusté à 1 litre (Treco and Lundblad, 2001).

Le milieu de croissance SPS est un milieu appauvri de pré-sporulation composé de : 0,5% extraits de levure, 1% bacto-peptone, 0,17% base azotée de levure sans acide aminé et sans sulfate d'ammonium, 0,5% sulfate d'ammonium, 1% acétate de potassium, 1,02% biphtalate de potassium, pH5.5, H₂0 ajusté à 1 litre (Wu and Lichten, 1994).

Le milieu KAc 1% est un milieu pauvre de sporulation composé de 1% acétate de potassium, (supplémenté ou non en acide aminé 0,001% suivant les auxotrophies portées par les souches, et en PPG2000 0,001%), H₂0 ajusté à 1 litre (Wu and Lichten, 1994).

### Protocole de sporulation

La souche diploïde a été striée sur une boite YPD à partir du stock conservé à -80°C. Après trois jours de croissance à 30°C, les cellules d'une colonie individuelle ont été déposées sur une boite contenant du milieu solide YPG. Après environ 6h d'incubation à 30°C, les cellules ont été suspendues dans 5mL de YPD liquide et incubées 24h à 30°C avec agitation (250 rpm). Cette pré-culture a été utilisée pour inoculer 50mL de milieu SPS à une concentration de 10⁵cellules/ml qui ont ensuite été incubés environ 18h à 30°C, jusqu'à atteindre 2-4.10⁷cellules/ml. Les cellules ont été lavées dans 50mL de milieu KAc 1% préchauffé à 30°C, puis centrifugées et resuspendues dans 100ml de milieu de sporulation KAc 1% préchauffé. Les cultures ont été incubées à 30°C sous agitation (250rpm) pendant des temps variables suivant les besoins de l'expérience. Des échantillons ont été prélevés à différents temps au cours de la sporulation (Wu and Lichten, 1994).

### Protocoles de « return-to-growth »

### Protocole 1 : isolement par étalement des cellules issues du RTG.

Après incubation pour un temps donné dans le milieu de sporulation, 1ml de culture a été prélevé. Les cellules ont été lavées dans 1ml d'H₂O (centrifugation 3min à 8000g), et resuspendues dans un volume final de 500µl d'H₂O. A ce stade, des cellules ont été déposées sur milieu YPD (environ 100 cellules /boite, incubation à 30°C) pour donner naissance à des colonies individuelles issues du processus de RTG.

### Protocole 2 : isolement par étalement des cellules recombinantes issues du RTG.

De manière alternative au protocole 1, les cellules prélevées au cours de la sporulation ont été déposées sur un milieu sélectif DO-Arginine (environ 10⁴ cellules / boite) afin de sélectionner les cellules recombinantes porteuses d'un allèle ARG4, permettant la croissance des cellules en absence d'arginine.

### Protocole 3 : isolement par micromanipulation des cellules RTG.

10µl de la suspension des cellules prélevées au cours de la sporulation ont été déposées dans la partie supérieure d'une boite de milieu YPD. 44 cellules individuelles non bourgeonnantes ont été déplacées au micromanipulateur sur la grille d'un microscope à dissection (Singer MSM System). Les boites ont été incubées à 30°C et régulièrement observées afin de suivre l'apparition de la 1^{ère} cellule fille et procéder à la séparation physique de la cellule mère et la cellule fille, soit environ 4h après leur prélèvement du milieu de sporulation. Les boites ont été incubées à 30°C pour obtenir des paires de colonies individuelles appelées « mère/fille ».

### Analyse phénotypique des cellules issues du RTG

### Test phénotypique du signe sexuel.

Pour tester le signe sexuel des cellules issues du RTG (souches AND1708, AND1709, AND1710, AND1711, AND1712, AND1720, AND1733, AND1734, AND1735, AND1736, AND1737, AND1738, AND1739, AND1740, AND2711, AND2907, AND2642, AND2652, AND2658), les cellules ont été déposées sur un milieu solide YPD et mises en présence de cellules de test haploïdes MATa his1 (ORT3805) ou MATα his1 (ORT3806). Le mélange de cellules a été incubé 24h à 30°C puis répliqué sur un milieu DOBA. L'absence de croissance des cellules sur ce milieu reflète l'incapacité des souches issue du RTG et des cellules de test à se croiser. Ceci est un indicateur phénotypique du caractère diploïde MATa/MATα des cellules issues du RTG.

### Test phénotypique de recombinaison.

Pour caractériser phénotypiquement la nature recombinante des cellules issues du RTG, la croissance des cellules a été examinée sur divers milieux sélectifs, rapporteurs du génotype des marqueurs portés par la souche parentale AND1702 (DO-Arginine, DO-Histidine, DO-Leucine, DO-Méthionine). Cette souche étant hétérozygote pour les marqueurs arg4-RV et arg4-Bgl empêchant la croissance des cellules sur un milieu dépourvu d'arginine (DO-Arginine), la formation par recombinaison de cellules RTG porteuses d'un allèle ARG4 permet aux cellules de croître sur ce milieu. La souche AND1702 portant également à l'état hétérozygote les allèles his3Δ200/HIS3, his4B::LEU2/HIS4 et met15Δ0/MET15, elle est entre autre de phénotype [HIS+ LEU+ MET+]. Le caractère recombinant des cellules RTG peut se révéler par la perte d'une de ces prototrophies ([his-]=his3Δ200/his3Δ200 ou his4B::LEU2/his4B::LEU2, [leu-]=HIS4/HIS4, [met-]= met15Δ0/met15Δ0).

### Test phénotypique sur les tétrades obtenues par sporulation des diploïdes RTG

Le caractère recombinant des cellules RTG peut également concerner la perte d'hétérozygotie d'un marqueur génétique sans changement de phénotype, par exemple une cellule RTG peut devenir MET15/MET15 par recombinaison, elle restera prototrophe pour la méthionine comme la souche diploïde parentale AND1702. Afin de déceler ces évènements, le phénotype de tétrades issues de diploïdes RTG a été analysé sur les milieux DO-Arginine, DO-Histidine, DO-Leucine et DO-Méthionine afin d'observer la ségrégation des marqueurs génétiques. Le type sexuel des cellules a aussi été déterminé selon la méthode décrite ci-dessus.

### Analyse du génotype des cellules issues du RTG par séquençage NGS

Les génomes des levures parentales ORT7219, ORT7221, et AND1702, et des cellules issues du RTG AND1708, AND1709, AND1710, AND1711, AND1712, AND1720, AND1733, AND1734, AND1735, AND1736, AND1737, AND1738, AND1739, AND1740, AND2711, AND2907, AND2642, AND2652, et AND2658, ont été séquencés par la méthode de séquençage NGS (*Next Generation Sequencing*) (Plateforme NGS Institut Curie, Paris, France). Pour les souches haploïdes ORT7219 et ORT7221, une banque de fragments d'ADN génomique a été réalisée et séquencée par la méthodologie « Paired-end » (50+35 nt) sur les séquenceurs SOLiD v4 suivant les protocoles du fournisseur « Life Technologies ». Pour les souches haploïdes AND1710-1A, AND1710-1B, AND1710-1C et AND1710-1D, une banque de fragment d'ADN génomique a été réalisée et séquencée par la méthodologie « Paired-end » (50+35 nt) sur les séquenceurs SOLiD v5500 suivant les protocoles du fournisseur « Life Technologies ». Pour les souches diploïdes (AND1702, AND1708, AND1709, AND1710, AND1711, AND1712, AND1720, AND1733, AND1734, AND1735, AND1736, AND1737, AND1738, AND1739 et AND1740) des banques «Mate-pair» (50+50nt) ont été réalisées à partir des préparations d'ADN génomiques et séquencées sur la plateforme sur les séquenceurs SOLiD v4 de la plateforme NGS de l'Institut Curie, suivant les protocoles du fournisseur « Life Technologies ». Les souches AND2711, AND2907, AND2642, AND2652 et AND2658 des banques « Paired-end » (100+100nt) ont été réalisées à partir des préparations d'ADN génomiques et séquençées sur la plateforme NGS de l'Institut Curie sur le séquenceur HiSeq 2500, suivant les protocoles du fournisseur « Illumina ».

### Analyse bioinformatique des données de séquençage NGS

Pour déterminer les polymorphismes apportés par les souches haploïdes parentales ORT7219 et ORT7221, les séquences issues du NGS ont été alignées sur la séquence du génome de référence S288C avec le logiciel Bioscope (Life Technologies). La version de la séquence de référence utilisée (R64) est disponible sur le site internet « Saccharomyces Genome Database » (SGD) (http://downloads.yeastgenome.org/sequence/S288C_reference/genome_releases/S288C_r eference_genome_R64-1-1_20110203.tgz). Les numéros d'entrée pour les 16 chromosomes et le génome mitochondrial sont : Chromosome (Chr.) I : NC_001133; Chr. II : NC_001134; Chr. III : NC_001135; Chr. IV : NC_001136; Chr. V : NC_001137; Chr. VI : NC_001138; Chr. VII : NC_001139; Chr. VIII : NC_001140; Chr. IX : NC_001141; Chr. X : NC_001142; Chr. XI : NC_001143; Chr. XII : NC_001144; Chr. XIII: NC_001145; Chr. XIV: NC_001146; Chr. XV: NC_001147; Chr. XVI: NC_001148, et Chr. mitochondrial: NC_001224. La liste et les coordonnées des polymorphismes SNPs (Single Nucleotide Polymorphisms) entre les souches parentales ORT7219 (S288C) et ORT7221 (SK1) a été établie avec l'outil « Find SNP » de Bioscope. Les séquences NGS de la cellule diploïde AND1702, des cellules issues du RTG et des quatre spores de la tétrade AND1710-1 ont été alignées avec le logiciel Lifescope (Life Technologies) sur la séquence du génome de référence SGD. Afin de déterminer le génotype de chacune des souches séquencées, les lectures chevauchant les positions polymorphiques appartenant à la liste de SNPs établie ont été sélectionnées avec l'outil « IntersectBED » du logiciel BEDTools (Quinlan et al., 2010). Puis chaque lecture a été associée avec le (ou les) polymorphisme qu'elle recouvre et la position du (ou des) polymorphismes dans la lecture a été calculée. La base à cette position a ensuite été extraite et comparée aux bases trouvées à cette position dans les souches parentales ORT7219 et ORT7221. A chaque position polymorphique, les lectures possédant l'allèle S288C, l'allèle SK1 ou un autre allèle ont été comptées. Le SNP a été déclaré mono-allélique d'origine S288C si l'allèle S288C est représenté dans plus de 82% des lectures. Il est déclaré mono-allélique d'origine SK1 si l'allèle SK1 est représenté dans plus de 68% des lectures et déclaré bi-allélique si l'allèle SK1 est représenté dans une proportion de 18 à 68% des lectures ou l'allèle S288C est représenté dans une proportion de 32 à 82% des lectures. La carte des positions polymorphique a ensuite été tracée à l'aide du logiciel R (http://www.r-project.org) pour chaque échantillon analysé. Le génotype de chaque position polymorphique a été renseigné par une couleur : noir pour mono-allélique S288C, gris moyen pour mono-allélique SK1 et gris clair pour bi-allélique. Pour tous les échantillons séquencés, la couverture par position a été calculée à l'aide de l'outil « genomeCoverageBed » de BEDTools. Les lectures 100nt + 100 nt issues du séquenceur Illumina ont été alignées sur le génome de référence (SGD) avec le logiciel BWA.

### Résultats

### Analyses phénotypiques

### Sélection des cellules RTG recombinantes

Six colonies indépendantes (souches AND1708, AND1709, AND1710, AND1711, AND1712, AND 1720) issues du RTG (parent AND1702, protocole 2 de sélection de cellules prototrophes pour l'arginine) ainsi que quatre couples de RTG « mère (M) et fille (F) » (AND1733(M)-AND1734(F), AND1735(M)-AND1736(F), AND1737(M)-AND1738(F) et AND1739(M)-AND1740(F)) issues du RTG (parent AND1702, protocole 3 d'isolement par micromanipulation des cellules méres/filles après la première division cellulaire) ont été retenues pour génotypage. La nature diploïde de ces souches a été confirmée par deux tests phénotypiques : l'absence de croisement avec des cellules haploïdes testrices de signe sexuel MATa (ORT3805) et MATa (ORT3806), et leur capacité à entrer en sporulation et former des tétrades à quatre spores viables (ci-dessous). Le phénotype des cellules RTG pour les marqueurs hétérozygotes (ARG, HIS, LEU, MET) est présenté dans le Tableau 3 ci-dessous.

**Tableau 3 : Phénotype des cellules RTG**

| Souche | DO-ARG | DO-HIS | DO-LEU | DO-MET | Testrice MATa | Testrice MATα |
|---|---|---|---|---|---|---|
| AND 1702 | - | + | + | + | - | - |
| AND1708 | + | - | + | + | - | - |
| AND 1709 | + | + | + | - | - | - |
| AND1710 | + | - | + | + | - | - |
| AND1711 | + | - | + | + | - | - |
| AND1712 | + | - | + | + | - | - |
| AND 1720 | + | + | + | - | - | - |
| AND1733 | + | - | + | + | - | - |
| AND1734 | - | - | - | + | - | - |
| AND1735 | - | - | + | + | - | - |
| AND1736 | - | + | + | + | - | - |
| AND1737 | - | + | - | + | - | - |
| AND1738 | - | - | + | + | - | - |
| AND1739 | - | + | + | + | - | - |
| AND1740 | - | - | + | + | - | - |
| AND2711 | - | - | + | + | - | - |
| AND2907 | - | - | + | + | - | + |
| AND2642 | - | + | + | - | - | - |
| AND2652 | - | - | + | + | - | - |
| AND2658 | - | - | + | + | - | - |

Aucune des cellules ne se croisent avec les testrices MATa et MATa, elles sont donc restées diploïdes. Sur l'ensemble des cellules issues de RTG séquencées, sept sont devenues prototrophes pour l'arginine (AND1708, AND1709, AND1710, AND1711, AND1712, AND1720 et AND1733), neuf sont devenues auxotrophes pour l'histidine (AND1708, AND1710, AND1711, AND1712, AND1733, AND1734, AND1735, AND1738, AND1740), deux sont devenues auxotrophes pour la leucine (AND1734 et AND1737) et deux sont devenues auxotrophes pour la méthionine (AND1709, AND1720). Ceci illustre la diversité phénotypique des cellules issues du RTG et est le résultat d'évènements de recombinaison.

### Identification du génotype des cellules RTG par analyse de tétrades

Pour observer la ségrégation des marqueurs génétiques des cellules RTG, celles-ci ont été mises en sporulation et dix tétrades ont été disséquées et analysées. Dans 13 cas sur 14 (AND1708, AND1709, AND1710, AND1712, AND1720, AND1733, AND1734, AND1735, AND1736, AND1737, AND1738, AND1739 et AND1740), des tétrades à quatre spores viables ont été obtenues, confirmant la nature diploïde de ces cellules RTG et indiquant l'absence de mutations létales dans le génome de ces diploïdes RTG. Pour AND 1711, les tétrades disséquées n'avaient que deux spores viables reflétant la présence d'une aneuploïdie détectée par l'analyse de la profondeur de couverture des lectures de séquence et confirmée par Southern blot. Cette aneuploïdie concerne la perte d'une copie de 170kb de l'une des extrémités du Chr. XVI, associée à un gain d'une copie de 110kb du Chr. V. La ségrégation des marqueurs génétiques dans les tétrades disséquées a permis de préciser le génotype des marqueurs de chaque diploïde comme indiqué dans le Tableau 1.

### Analyse bioinformatique

Le génome des haploïdes parentaux ORT7219 et ORT7221, le génome du diploïde hybride AND1702 et le génome des cellules RTG AND1708, AND1709, AND1710, AND1711, AND1712, AND1720, AND1733, AND1734, AND1735, AND1736, AND1737, AND1738, AND1739 et AND1740 ont été séquencés en NGS et les lectures analysées suivant les méthodes décrites ci-dessus.

### Analyse primaire des données de séquençage

Pour chaque échantillon, plus de 60 millions de lectures NGS ont été obtenues avec une couverture (nombre de lectures par position) homogène sur l'ensemble du génome supérieure à 100X. La couverture moyenne par échantillon est donnée dans le Tableau 4.

**Tableau 4 : Indice moyen de couverture obtenu pour chaque échantillon séquencé, après retrait des duplicats de PCR**

| Echantillon | Méthode d'alignement | Indice moyen de couverture |
|---|---|---|
| ORT7219 | Bioscope | 192X |
| ORT7221 | Bioscope | 141X |
| AND 1702 | Lifescope | 43X |
| AND1708 | Lifescope | 95X |
| AND 1709 | Lifescope | 174X |
| AND1710 | Lifescope | 181X |
| AND1711 | Lifescope | 186X |
| AND1712 | Lifescope | 189X |
| AND 1720 | Lifescope | 179X |
| AND1733 | Lifescope | 16X |
| AND1734 | Lifescope | 27X |
| AND1735 | Lifescope | 80X |
| AND1736 | Lifescope | 69X |
| AND1737 | Lifescope | 53X |
| AND1738 | Lifescope | 28X |
| AND1739 | Lifescope | 15X |
| AND1740 | Lifescope | 21X |
| AND1710-1A | Lifescope | 124X |
| AND1710-1B | Lifescope | 111X |
| AND1710-1C | Lifescope | 90X |
| AND1710-1D | Lifescope | 120X |
| AND2711 | BWA | 90X |
| AND2907 | BWA | 89X |
| AND2642 | BWA | 89X |
| AND2652 | BWA | 90X |
| AND2658 | BWA | 91X |

### Identification des polymorphismes différenciant les haploïdes parentaux

L'analyse des séquences NGS de la souche ORT7219 contre la séquence de référence SGD a conduit à identifier 115 SNPs. L'analyse des séquences NGS de la souche ORT7221 contre la séquence de référence SGD a conduit à identifier 65 134 SNPs. Parmi ceux-ci, 63 901 SNPs ont été retenus pour le génotypage des lectures NGS du diploïde hybride AND1702 et des cellules issues du RTG. La distance physique entre les SNPs varie entre 2 et 38 036 nucléotides avec une médiane de 96 nucléotides et une moyenne de 187 nucléotides.

### Génotype des cellules RTG

Tous les polymorphismes parentaux ont été retrouvés dans les cellules séquencées. Chaque souche RTG s'est révélée porteuse de SNPs mono-alléliques et de SNPs bi-alléliques en nombre variable. Les résultats reportés dans le Tableau 5 ci-dessous montrent que 61 à 89% des SNPs sont bi-alléliques confirmant le caractère diploïde de ces cellules issues du RTG. Les autres SNPs sont mono-alléliques, correspondant dans 6 à 26% des cas à l'allèle S288C et correspondant dans 1 à 18% des cas à l'allèle SK1, c'est-à-dire qu'en moyenne 17% des SNPs se retrouvent à l'état mono-allélique. La présence d'un seul allèle à ces positions mono-alléliques indique une perte d'hétérozygotie (*Loss of heterozygosity* ou LOH) qui peut refléter soit la présence de deux chromosomes homologues porteurs du même allèle soit d'une perte de la région chromosomique sur l'un des chromosomes homologues. L'analyse de la couverture moyennée sur 1kb de chaque chromosome montre que les régions mono-alléliques et les régions hétéro-alléliques ont un indice de couverture homogène, ce qui est en faveur de l'hypothèse de deux chromosomes homologues homozygotes.

**Tableau 5 : Pourcentage de perte d'hétérozygotie dans les différentes souches RTG**

| souche | Durée d'incubation en KAc | Proportion de positions mono-alléliques S288C (%) | Proportion de positions mono-alléliques SK1 (%) | Proportion de position bi-alléliques (%) |
|---|---|---|---|---|
| AND1708 | 6h | 25,1 | 2,9 | 71,8 |
| AND 1709 | 5h | 13,3 | 13,4 | 73,1 |
| AND1710 | 5h | 13,4 | 12,0 | 74,3 |
| AND1711 | 5h | 14,5 | 13,7 | 71,6 |
| AND1712 | 5h | 8,9 | 6,7 | 84,3 |
| AND 1720 | 5h | 12,5 | 9,2 | 78,1 |
| AND1733 | 5h | 17,7 | 8,8 | 73,5 |
| AND1734 | 5h | 9,5 | 16,6 | 73,9 |
| AND1735 | 5h | 5,4 | 4,4 | 90,2 |
| AND1736 | 5h | 4,1 | 5,4 | 90,6 |
| AND1737 | 8h | 6,5 | 0,2 | 93,3 |
| AND1738 | 8h | 0,2 | 6,4 | 93,3 |
| AND1739 | 8h | 8,6 | 3,7 | 87,6 |
| AND1740 | 8h | 4,0 | 8,2 | 87,8 |
| AND2711 | 4h | 21,5 | 10,9 | 67,6 |
| AND2907 | 6h | 26,8 | 19,7 | 53,5 |
| AND2642 | 8h | 8,7 | 6,9 | 84,4 |
| AND2652 | 8h | 11,6 | 9,0 | 79,3 |
| AND2658 | 8h | 5,5 | 1,2 | 93,1 |

La cartographie de l'état des SNPs (bi-allélique, mono-allélique d'origine S288C ou mono-allélique d'origine SK1) des cellules RTG analysées est illustrée dans les Figures 1 et 2. Les cellules isolées selon le protocole 2 ci-dessus sont illustrées dans la Figure 1. Les cellules isolées selon le protocole 3 ci-dessus sont illustrées dans la Figure 2 (où les deux cellules mère et fille provenant du même événement de RTG sont regroupées sur la même figure, afin de faciliter la comparaison des génotypes deux à deux).

Le génotype de ces cellules est très différent. Dans la majorité des cas, chaque chromosome est composé de régions bi-alléliques et mono-alléliques de tailles variables impliquant une ou un grand nombre de positions SNPs (allant de 1 nucléotide à près de 700 kb), d'origine S288C et/ou SK1. L'agencement des régions bi- et mono-alléliques, révélant l'existence d'une recombinaison localisée aux points de jonction, est différent suivant les cellules. En tenant compte uniquement des régions mono-alléliques d'au moins 20kb, le nombre de jonctions recombinantes par chromosome varie de 0 à 7 et le nombre total estimé par cellule est de 34, 53, 15, 38, 7, 18, dans les souches RTG AND1708 (Fig. 1A), AND1709 (Fig. 1B), AND1710 (Fig. 1C), AND1711 (Fig. 1D), AND1712 (Fig. 1E), AND1720 (Fig. 1F), et de 45, 6, 3, 11 dans les couples mère-filles AND1733-AND1734 (Fig. 2A), AND1735-AND1736 (Fig. 2B), AND1737-AND1738 (Fig. 2C) et AND1739-AND1740 (Fig. 2D). On remarque que (i) les SNPs localisés près des centromères restent bi-alléliques, ce qui confirme la ségrégation mitotique des chromatides soeurs en RTG, et (ii) le génotype des cellules mère et fille est complémentaire, étant identique dans les régions bi-alléliques et d'allèles opposés dans les régions mono-alléliques. Contrairement à l'isolement de cellules RTG individuelles (protocoles 1 et 2), la méthode d'isolement des couples mère et fille (protocole 3) a l'avantage de permettre l'analyse des phénotypes dans un contexte homogène pour les SNPs bi-alléliques.

### Confirmation du génotype de la souche AND1710 par séquençage d'une tétrade

Quatre spores provenant d'une tétrade issue de la sporulation du diploïde RTG AND1710 ont été séquencées et le génotype des positions de polymorphisme a été déterminé. Le génotype de polymorphisme des 4 spores (A, B, C, D) de la tétrade est illustré dans la figure 3.

Pour les régions mono-alléliques du diploïde AND1710, on observe que les quatre spores ont le même allèle d'origine S288C ou SK1. Les régions bi-alléliques du diploïde ségrègent dans 2 spores portant l'allèle S288C et 2 spores portant l'allèle SK1, ce qui confirme la nature hétérozygote du diploïde dans ces régions. Les 15 jonctions recombinantes du diploïde RTG AND1710 sont confirmées. De plus, au niveau de 4 courtes régions mono-alléliques du diploïde AND1710, des évènements de recombinaison supplémentaires (chr. VII, chr. VIII et chr. IX (2 cas)) associés à des conversions géniques ont pu être identifiés. Les autres évènements de recombinaison ont eu lieu au cours de la méiose aboutissant à la formation de cette tétrade et le génotype recombiné n'était pas présent dans le diploïde RTG parental.

### Réitération du processus RTG

Afin d'évaluer si le processus RTG pouvait être réitéré pour accroître la diversification génétique, les inventeurs ont réalisé deux cycles successifs de RTG à partir de la souche AND1735 (***Fig. 4A***) déjà issue d'un premier cycle de RTG du diploïde parental AND1702 puis analysé par séquençage l'évolution des marqueurs SNPs. Lors du second cycle RTG, la cellule AND2711 a subi un autre cycle de recombinaison qui se manifeste par une diminution du taux global d'hétérozygotie (67,6% au lieu de 90,2%) et l'apparition de nouvelles régions mono-alléliques de fond génétique S288C ou SK1 (***Fig. 4B***)*.* Puis, lors du troisième cycle RTG, la cellule AND2907 a subi un autre cycle de recombinaison qui se manifeste par une diminution supplémentaire du taux global d'hétérozygotie (53,5% au lieu de 67,6%) et l'apparition de nouvelles régions mono-alléliques de fond génétique S288C ou SK1 (***Fig. 4C***, ***Tableaux 5 et 6***)*.* Le processus RTG peut donc être réitéré pour accroître de manière séquentielle la diversification génétique des cellules diploïdes.

### RTG dans les cellules diploïdes AND2248 déficientes en sporulation

Afin d'évaluer si le processus RTG pouvait être utilisé pour recombiner le génome de cellules diploïdes stériles, les inventeurs ont construit une souche diploïde (AND2248) de fond génétique hybride S288C/SK1 mais porteuse à l'état homozygote d'une délétion du gène NDT80. L'inactivation du gène NDT80 conduit à l'absence de formation des spores (d'où le phénotype de stérilité) mais n'empêche pas l'entrée des cellules diploïdes en prophase de méiose. Ces cellules arrêtent leur progression en méiose à un stade postérieur à la formation des cassures double brin de l'ADN Spo 11-dépendantes mais avant l'étape de division réductionnelle des chromosomes (M1) (Chu & Herskowitz, 1998). Les cellules diploïdes *ndt80Δ*/*ndt80Δ* ainsi arrêtées en prophase de méiose restent viables et sont capables de retourner en croissance végétative grâce au processus RTG (Dayani et al., 2011). Trois souches isolées après RTG (protocole 3) de la souche mutante AND2248 ont été séquencées. La cartographie des SNPs de ces souches (AND2642, AND2652 et AND2658) est illustrée respectivement dans les fig. 5A-C. Leurs génotypes sont recombinés et distincts (Tableau 5). Respectivement, les degrés d'hétérozygosité sont de 84,4%, 79,3% et 93,1%, le reste du génome porte des positions mono alléliques d'origine S288C ou SK1 et le nombre total de jonctions recombinantes est de 12, 24 et 5 par cellule. La méthode RTG est donc applicable à des souches stériles capables de former les cassures double-brin naturelles Spo11-dépendantes.

### Analyse de la variabilité du génotype des cellules RTG.

Les cellules RTG étant généralement diploïdes (sauf AND1711), la diminution du pourcentage d'hétérozygosité par cellule s'accompagne de l'apparition de régions homozygotes de génotype S288C ou SK1. La variation du pourcentage d'hétérozygosité et d'homozygosité des 19 souches issues d'un processu RTG (Tableau 5) est illustrée dans la Figure 6. Le pourcentage d'hétérozygosité (génotype bi-allélique S288C+SK1) varie entre 93,3% (AND1738 et AND1739 issues du protocole 3) et 53,5% (AND2907) issue de trois cycles de RTG. La proportion des régions homozygotes S288c varie entre 0,2% (AND1738) et 26,8% (AND2907). La proportion des régions homozygotes SK1 varie entre 0,2% (AND1737) et 19,7% (AND2907). La taille de ces régions homozygotes peut être courte en n'impliquant que quelques marqueurs SNPs adjacents ou très grandes en impliquant de grandes régions chromosomiques. Le nombre total de jonctions recombinantes par cellule RTG, estimé en ne tenant compte que des régions recombinées dont la longueur est d'au moins de 20kb, varie entre 3 (AND1737) et 53 (AND1709). La méthode RTG permet donc de créer une population de cellules diversement recombinées, étant simultanément porteuses de régions hétérozygotes et homozygotes pour l'un ou l'autre des génotypes du parent hybride et de tailles variables, et cela aussi bien dans les cellules fertiles et stériles.

### Exemple de cartographie du caractère d'auxotrophie pour la méthionine et la leucine

Afin d'évaluer les performances de la méthode RTG pour identifier et localiser un caractère phénotypique simple, les inventeurs ont analysé le génotype et le phénotype des cellules RTG pour le caractère de croissance en absence de méthionine. La souche S288C possède une délétion du gène MET15 (met15Δ0). Ainsi, le diploïde parental porte le caractère bi-allélique MET15/met15Δ0. Un diploïde recombinant auxotrophe pour la méthionine doit posséder des marqueurs mono-alléliques d'allèle S288C aux alentours du locus MET15 (chr. XII). A l'inverse, les diploïdes prototrophes pour la méthionine peuvent porter des marqueurs mono-alléliques d'allèle SK1 ou bi-alléliques aux alentours du locus MET15, mais jamais des marqueurs mono-alléliques d'allèle S288C. Ainsi, il est possible de cartographier les régions chromosomiques associées au phénotype en recherchant les positions de SNPs où un ou deux allèles parmi {S288C - SK1 - bi-allélique} sont trouvé(s), de façon spécifique et exclusive, associé(s) à l'un ou à l'autre des phénotypes. Pour cela, les inventeurs ont examiné la croissance des cellules parentales haploïdes ORT7219 et ORT7221, du diploïde hybride AND1702 et des cellules RTG AND1708, AND1709, AND1710, AND1711, AND1712, AND1720, AND1733, AND1734, AND1735, AND1736, AND1737, AND1738, AND1739 et AND1740 sur le milieu DO-méthionine. Conformément à leurs génotypes (Tableau 1), les cellules diploïdes bi-alléliques *MET15*/*met15Δ0* (parent hybride AND1702, RTG AND1708, AND1712, AND1733, AND1734, AND1735, AND1736, AND1737, AND1738, AND1739, AND1740) et les diploïdes mono-allélique MET15/MET15 (RTG AND1710 et AND1711) étaient capables de croître en absence de méthionine tandis que les cellules diploïdes mono-alléliques met15Δ0/met15Δ0 (RTG AND1709 et AND1720) étaient incapables de croître en absence de méthionine. Pour déterminer la région du génome portant ce caractère, les inventeurs ont regroupé les cellules RTG en deux catégories : prototrophes (RTG AND1708, AND1710, AND1711, AND1712, AND1733, AND1734, AND1735, AND1736, AND1737, AND1738, AND1739, AND1740) et auxotrophes (RTG AND1709 et AND1720), puis comparé leurs génotypes afin d'identifier les régions du génome pour lesquelles les allèles trouvés chez les individus prototrophes sont trouvés de façon exclusive dans cette catégorie, de même que les allèles trouvés chez les individus auxotrophes sont trouvés de façon exclusive dans cette catégorie. Avec seulement 2 échantillons dans la catégorie auxotrophe, cette méthode identifie le caractère dans un nombre très restreint de régions candidates (6 régions et quelques SNPs isolés) (***Fig.* 7A**) incluant la région recherchée du gène MET15. A l'aide d'une simulation bioinformatique, où les deux échantillons homozygotes MET15/MET15 (AND1710 et AND1711) ont été artificiellement rendus homozygotes *met15Δ0*/*met15Δ0* par inversion des allèles des locus mono-alléliques, les inventeurs ont pu montrer qu'en équilibrant le nombre d'échantillon dans les deux catégories, on pouvait aisément réduire le nombre de régions candidates à 1, incluant le gène MET15. Dans cet exemple, la région identifiée est d'environ 40 kb. L'étude d'un plus grand nombre de cellules RTG a pour effet de réduire la taille de la région candidate.

De la même façon, les inventeurs ont analysés le génotype et le phénotype des cellules RTG pour le caractère de croissance en absence de leucine. Les deux souches parentales possèdent une mutation du locus LEU2 (leu2Δ0 pour le parent S288c et leu2::HisG pour le parent SK1, Chr. III). Cependant le parent SK1 possède une copie sauvage du gène LEU2 insérée dans un locus voisin (marqueur his4B::LEU2 sur le chromosome III). Ceci conduit à l'obtention, après RTG, de diploïdes mono-alléliques his4B::LEU2/his4B::LEU2 (AND1708, AND1733, AND1738), de diploïdes bi-alléliques his4B::LEU2/HIS4 (AND1709, AND1710, AND1711, AND1712, AND1720, AND1735, AND1736, AND1739, AND1740), constituant le groupe des cellules RTG prototrophes pour la leucine, et de diploïdes mono-alléliques HIS4/HIS4 (AND1734, AND1737), constituant le groupe des auxotrophes pour la leucine. Ici, avec seulement 2 échantillons dans la catégorie des auxotrophes, la cartographie du phénotype de croissance sur le milieu DO-leucine aboutit à l'identification de seulement deux régions candidates (***Fig. 7B***), incluant la région recherchée des gènes LEU2 et HIS4.

Il s'agit d'exemples de cartographie d'un caractère mendélien par séquençage d'un faible nombre de cellules RTG, applicable pour les hybrides stériles.

### Exemple d'amélioration d'un caractère quantitatif par la méthode RTG

Afin d'évaluer les performances de la méthode RTG pour identifier et localiser des caractères quantitatifs, les inventeurs ont examiné la croissance à la température de 30°C et 40°C des cellules parentales haploïdes ORT7219 et ORT7221, du diploïde hybride AND1702 et des cellules RTG AND1708, AND1709, AND1710, AND1712 et AND1720, AND1733, AND1734, AND1735, AND1736, AND1737, AND1738, AND1739 et AND1740. Les résultats illustrés dans la figure 8 montrent que les cellules parentales haploïdes ORT7235 et ORT7236 poussent peu à la température de 40°C. Au contraire, la cellule hybride AND1702 manifeste le phénomène de vigueur hybride (hétérosis) car elle croît mieux à cette température que chacun des parents. Les cellules issues du RTG manifestent un phénotype variable, en poussant plus ou moins bien à 40°C. En particulier, les cellules RTG AND1708, AND1710, AND 1712, AND1735, AND1736 et AND1737 sont plus thermo-tolérantes que les cellules parentales et au moins aussi thermo-tolérantes que la cellule diploïde hybride AND 1702. Egalement, les inventeurs ont examiné la croissance des cellules en présence d'arsénite de sodium (NaAsO₂ 1,5mM). La croissance des cellules RTG est variable. Par exemple, les cellules AND1736 et AND1737 sont plus résistantes que la cellule diploïde parentale AND1702, tandis que les cellules AND1735 et AND1738 sont plus sensibles.

Il s'agit de deux exemples d'amélioration d'un caractère quantitatif d'intérêt. Le nombre et la localisation des marqueurs polymorphiques causaux pourront être déduits de l'analyse comparative du génotype des cellules obtenu par séquençage.

### REFERENCES BIBLIOGRAPHIQUES

Acquaviva L., et al., (2013). Science, 339, 215-218
Albers et al., (1996),.Applied and Environmental Biology, 62:3187-3195
Becker and Guarente, Methods Enzymol., (1991)194,182-7.
Ben-Ari et al., (2006). PLoS Genet. 2(11): e195.
Chu and Herskowitz (1998) Mol. Cell 1, 685-696.
Dayani et al., (2011). PloS Genet. 7(5):e1002083
De Massy et al., (1994). Proc. Nat. Acad. Sci USA, 91, 11929-11933
Esposito and Esposito (1974). Proc. Nat. Acad. Sci USA,71 (8), 3172-3176
Greig (2007). PLoS Genet., 3(2): e21.
Herman and Roman (1963). Genetics, 48,255-261
Honigberg and Esposito (1994). Proc. Nat. Acad. Sci USA, 91, 6559-6563.
Honigberg and Purnapatre (2003). J. Cell Sci. 116, 2137-2147
Kane and Roth (1974). Bacteriol, 118(1), 8-14.
Kassir and Simchen (1991). Meth. Enzymol. 194, 94-110.
Keeney (2001). Curr. Top. Dev. Biol. 52, 1-53.
Mortimer and Johnston (1986). Genetics, 113(1), 35-43.
Nicolas et al., (1989). Nature, 338, 35-39
Quinlan et al., (2010). Bioinformatics, 15;26(6), 841-2.
Rainieri et al., (1999). S. Afr. J. Enol. Vitic., Vol. 20, No.2
Rocco et al., (1992). Proc. Nat. Acad. Sci USA, 89, 12068-72.
Smith and Nicolas (1998). Curr. Opin. Genet. Dev. 8(2), 200-211
Sherman (1991). Meth. Enzymol. 194, 3-21
Treco and Lundblad (2001) Current Protocol in MolecularBiology Chapter 13:Unit13.1.
Voth et al., (2003). YEAST, 20(11), 985-93.
Wu and Lichten (1994). Science 263, 515-518.
Zenvirth et al. (1997). Genes to Cells 2, 487-498

## Revendications

1. Procédé pour diminuer le taux d'hétérozygotie d'une levure, comprenant :
a) le transfert de la levure d'un milieu riche à un milieu de sporulation ;
b) l'incubation des levures dans le milieu de sporulation durant un temps suffisant pour induire la formation des cassures double-brin Spo 11-dépendantes ;
c) la mise en contact des levures avec une source de carbone et d'azote avant la ségrégation réductionnelle des chromosomes de la première division de méiose afin d'obtenir des levures recombinées ; et
d) la récupération des levures recombinées ; et
lesdites levures recombinées présentant un taux d'hétérozygotie inférieur à celui de la levure de l'étape a).

2. Procédé selon la revendication 1, **caractérisé en ce que** les étapes a) à d) sont répétées au moins une fois à partir d'une ou plusieurs levures recombinées.

3. Procédé selon la revendication 1 ou 2, dans laquelle les levures recombinées récupérées à l'étape d) sont conservées sous forme de banques de levures avant d'être criblées ou sélectionnées.

4. Utilisation du processus RTG pour diminuer le taux d'hétérozygotie d'une levure, ledit processus RTG étant induit par le transfert de la levure d'un milieu riche à un milieu de sporulation, l'incubation des levures dans le milieu de sporulation durant un temps suffisant pour induire la formation des cassures double-brin Spo11-dépendantes, et la mise en contact des levures avec une source de carbone et d'azote avant la ségrégation réductionnelle des chromosomes de la première division de méiose.

5. Procédé selon l'une quelconque des revendications 1 à 3 ou utilisation selon la revendication 4, **caractérisé en ce que** ladite souche de levure présente un degré de ploïdie supérieur ou égal à 2.

6. Procédé selon l'une quelconque des revendications 1 à 3 ou utilisation selon la revendication 4 ou 5, **caractérisé en ce que** ladite levure est une souche de levure hybride.

7. Procédé selon l'une quelconque des revendications 1 à 3, 5 et 6 ou utilisation selon l'une quelconque des revendications 4 à 6, **caractérisé en ce que** ladite souche de levure est une souche stérile.

8. Procédé selon l'une quelconque des revendications 1 à 3 et 5 à 7 ou utilisation selon l'une quelconque des revendications 4 à 7, **caractérisé en ce que** la souche de levure est une souche d'intérêt industriel, de préférence destinée à l'industrie alimentaire, à la production de biocarburant, à la production de molécules d'intérêt ou à la dégradation de la cellulose ou de biomasse lignocellulosique.

9. Procédé selon l'une quelconque des revendications 1 à 3 et 5 à 8 ou utilisation selon l'une quelconque des revendications 4 à 8, **caractérisé en ce que** la souche de levure est un organisme non génétiquement modifié.

10. Procédé selon l'une quelconque des revendications 1 à 3 et 5 à 9 ou utilisation selon l'une quelconque des revendications 4 à 9, **caractérisé en ce que** les levures recombinées sont des organismes non génétiquement modifiés.

11. Procédé selon l'une quelconque des revendications 1 à 3 et 5 à 10 ou utilisation selon l'une quelconque des revendications 4 à 10, **caractérisé en ce que** la souche de levure appartient à l'espèce *Saccharomyces cerevisiae* ou est un hybride obtenu à partir de *Saccharomyces cerevisiae.*

12. Procédé selon l'une quelconque des revendications 1 à 3 et 5 à 11 ou utilisation selon l'une quelconque des revendications 4 à 11, **caractérisé en ce que** le milieu de sporulation ne comprend ni source de carbone fermentescible, ni source d'azote.

13. Procédé selon l'une quelconque des revendications 1 à 3 et 5 à 12 ou utilisation selon l'une quelconque des revendications 4 à 12, **caractérisé en ce que** la mise en contact des levures avec une source de carbone et d'azote avant la ségrégation réductionnelle des chromosomes de la première division de méiose se fait en transférant les levures dans un milieu riche.


SEQUENCE LISTING

<110>  Institut Curie
       INSERM (Institut National de la Santé et de la Recherche
       Médicale)
       Centre National de la Recherche Scientifique
       Université Nice Sophia Antipolis
       Sorbonne Université

<120>  PROCEDE D'AMELIORATION DE SOUCHES DE LEVURE

<130>  B1507EPPC1

<160>  2

<170>  PatentIn version 3.3

<210>  1
<211>  20
<212>  DNA
<213>  Artificial

<220>
<223>  Amorce sens ARG4

<400>  1
tactcattgg cagaatcccg                                                   20


<210>  2
<211>  20
<212>  DNA
<213>  Artificial

<220>
<223>  Amorce antisens ARG4

<400>  2
cgcttgagag gaagattagc                                                   20